# EUROPEAN PATENT APPLICATION

(11) **EP 4 276 174 A1**
(43) Date of publication of application: **15.11.2023**
(21) Application number: 22305686.2
(22) Date of filing: 09.05.2022
(51) Int. Cl.: C12N 5/0783, A61K 35/17, C12N 5/0789, C12N 9/12, C12N 15/63, C12N 15/90

(54) **GENE THERAPY FOR THE TREATMENT OF ACTIVATED PI3KINASE DELTA SYNDROME TYPE 1 (APDS1)**

(71) Applicant: Cellectis S.A., 75013 Paris (FR); Institut National de la Santé et de la Recherche Médicale (INSERM), 75013 Paris (FR); Assistance Publique-Hôpitaux de Paris (APHP), 75004 Paris (FR); Université Paris Cité, 75006 Paris (FR); Fondation Imagine, 75015 Paris (FR)
(72) Inventor: JUILLERAT, Alexandre, NEW YORK, 10016 (US); DUCHATEAU, Philippe, 75013 PARIS (FR); VALTON, Julien, 75013 PARIS (FR); BOYNE, Alex, NEW YORK, 10016 (US); KRACKER, Sven, 75013 PARIS (FR); CAVAZZANA, Marina, 75015 PARIS (FR); POGGI, Lucie, 75015 PARIS (FR)
(74) Representative: Santarelli

(57) **Abstract**

The present invention generally relates to the field of genome engineering (gene editing), and more specifically to *ex vivo* gene therapy for the treatment of Activated PI3kinase Delta Syndrome type 1 (APDS1) related to *PIK3CD* gene. Particularly, the present invention pertains to the treatment of *PIK3CD* deficiency in hematopoietic stem cells (HSCs) and/or T-cells. The present invention provides means and methods for genetically modifying HSCs and/or T-cells involving gene editing reagents, such as TALE-nucleases, that specifically target an endogenous *PIK3CD* locus, at least in the *PIK3CD* allele comprising at least one APDS1-associated mutation, thereby allowing the restoration of the normal cellular phenotype. The present invention also provides engineered PIK3CD-edited HSCs and engineered PIK3CD-edited T-cells comprising at least one exogenous sequence comprising a nucleic acid sequence encoding a functional PI3Kδ protein which is integrated in said HSCs' or T-cells' genome into a *PIK3CD* locus, in a non-functional *PIK3CD* allele, resulting in the expression of a functional PI3Kδ polypeptide. The present invention further provides populations of cells comprising said engineered HSCs or T-cells, pharmaceutical compositions comprising said engineered cells or populations of cells, as well as their use in gene therapy for the treatment of APDS1.

## Description

### FIELD OF THE INVENTION

The present invention generally relates to the field of genome engineering (gene editing), and more specifically to gene therapy for the treatment of Activated PI3kinase Delta Syndrome type 1 (APDS1) related to *PIK3CD* gene. Particularly, the present invention pertains to the correction of PI3K deficiency in hematopoietic stem cells (HSCs) and T-cells originating from a patient suffering from APDS1. The present invention provides means and methods for genetically modifying HSCs and T-cells involving gene editing reagents, such as TALE-nucleases, that specifically target an endogenous *PIK3CD* locus, in particular at least a non-functional endogenous *PIK3CD* gene comprising at least one APDS1-associated mutation, thereby allowing the restoration of a normal cellular phenotype. The present invention also provides engineered PIK3CD-edited- HSCs or T-cells comprising an exogenous sequence comprising a nucleic acid sequence encoding a functional PI3Kδ catalytic subunit p110δ, which is integrated in said HSCs' or T-cells' genome at least into a non-functional allele of a *PIK3CD* endogenous locus, resulting in the expression of a functional PI3Kδ polypeptide. The present invention further provides populations of cells comprising said engineered HSCs or T-cells, pharmaceutical compositions comprising said engineered HSCs or T-cells, or populations of cells, as well as their use in gene therapy for treatment of APDS1 related to *PIK3CD* gene.

### BACKGROUND OF THE INVENTION

Activated PI3kinase Delta Syndrome type 1 (APDS1) is also known as PASLI (Lucas et al. (2014) Nature immunology, 15(1): 88-97), also called PASLI-CD. is a primary immunodeficiency caused by heterozygous gain of function mutations affecting the *PIK3CD* gene. A consequence of these autosomal dominant mutations is hyper activated PI3kinase δ signaling in immune cells leading to a combined immunodeficiency.

Class IA PI3Kinase (PI3K) molecules are composed of a p110 catalytic subunit and a regulatory subunit. The function of class IA PI3Ks is to convert phosphatidylinositol 4,5-bisphosphate into phosphatidylinositol 3,4,5-trisphosphate (PIP3), an important phospholipid secondary messenger (Zvelebil et al. (1996) Philosophical Transactions of the Royal Society of London. Series B, Biological Sciences, 351(1336): 217-223). The genes *PIK3CA, PIK3CB* and *PIK3CD* encode for the class IA PI3K catalytic subunits p110α, p110β, and p110δ, respectively. P110δ is predominantly expressed in leukocytes (Vanhaesebroeck et al. (1997) Proceedings of the National Academy of Sciences of the United States of America, 94(9): 4330-4335).

Activation of the PI3Kδ pathway through several membrane receptors, including T cell receptor/B cell receptor, cytokine receptors and co-stimulatory membrane molecules, lead to phosphorylation of downstream molecules, among them AKT and ribosomal protein S6 (Hawkins et al. (2006) Biochemical Society Transactions, 34(Pt 5): 647-662)

Hyperactivation of the PI3Kδ pathway leads to a complex immunodeficiency: in 2013-2014, two independent reports identified heterozygous missense mutations in *PIK3CD:* E1021K (Angulo et al. (2013) Science, 342(6160): 866-871) and N334K, E525K (Lucas et al. (2014) Nature immunology, 15(1): 88-97) using whole-exome sequencing. The authors showed that these mutations were autosomal dominant gain-of-function and led to increased PI3Kδ signaling responsible for a lymphoproliferation-associated primary combined immunodeficiency syndrome: APDS1. Later, further APDS1-causing gene modifications were described (E81K, G125D (Heurtier et al. (2017) Haematologica, 102(7): e278-e281 ; Takeda et al. (2017) J. Allergy Clin. Immunol. 140(4): 1152-1156)), R405C (Rae et al. (2017) Clinical Immunology (Orlando, Fla.), 181: 29-31). Higher levels of phosphorylated AKT and reduced levels of Foxo 1 were also observed (Lucas *et al.* (2014), supra). Addition of p110δ inhibitor rescued this phenotype providing a further proof of increased PI3Kδ signaling.

Clinical features of APDS1 are highly variable, even in the same family, and range from profound combined immunodeficiency (associated to lymphoproliferation, severe bacterial and viral infections from childhood) to isolated humoral defects. In several cohort studies, nearly all APDS1 patients were described as suffering from early-onset, recurrent and severe respiratory infections including sinusitis, nasopharyngitis, tonsillitis, otitis media, mastoiditis, pneumonia and pulmonary empyema (Crank et al. (2014) Journal of clinical immunology, 34(3): 272-276; Condliffe and Chandra (2018) Frontiers in Immunology, 9: 338). They also presented autoimmune manifestations, occurring in most cases after the first decade of life. Additionally, APDS1 predisposes to different types of B cell lymphoma especially classical Hodgkin lymphoma, diffuse large B cell lymphoma and marginal zone B cell lymphoma (Crank *et al.* (2014) supra; Kracker *et al.* (2014) supra; Lucas *et al.* (2014) supra).

Immunophenotyping of APDS1 patients revealed CD4+ T-cells lymphopenia, with a decrease in naive CD4+ and CD8+ T-cells count as well as an increase in effector memory CD8+ T-cells count resulting in normal to high counts of CD8⁺ T-cells and a subsequent inverted CD4/CD8 ratio (Lucas *et al.* (2014), supra).

Currently, treatments for APDS1 mainly consist in prophylactic measures including long term antibiotics and Immunoglobulins replacement therapy (Coulter et al. (2017) The Journal of Allergy and Clinical Immunology, 139(2): 597-606). Rapamycin (Sirolimus) treatment targeting mammalian target of rapamycin (mTOR), a downstream signaling component of the PI3K δ-signaling and a regulator of cell proliferation was tested with some success since it lowered lymphoproliferation (Maccari et al. (2018) Frontiers in Immunology, 9: 543). Two studies using selective PI3Kδ inhibitors, Leniolisib (Rao et al. (2017) Blood, 130(21): 2307-2316) and Seletalisib (Diaz et al. (2020) Journal of Immunology, 205(11): 2979-2987) reported reduction in lymphadenopathy and normalization of immune B cell subpopulations. However, these treatments may result in side effects outside of the immune system: proteinuria was observed after rapamycin administration and liver toxicity reported for Seletalisib (Diaz *et al.* (2020) supra). Additionally, PI3Kδ inhibitors harbor the risk to increase genomic instability in B cells by increasing AID expression and consequently mutations in off-target genes, other than Immunoglobulin sites, as shown with Idelalisib in murine and human B cells (Compagno et al. (2017) Nature, 542(7642): 489-493).

Massive lymphoproliferation associated to life-threatening progressive combined immunodeficiency and autoimmunity are indications for undergoing hematopoietic stem cell transplantation (Nademi et al. (2017) The Journal of Allergy and Clinical Immunology, 139(3): 1046-1049). HSC transplantation (HSCT) appears as the only cure for APDS1. None of the surviving HSCT patients required Immunoglobulins replacement therapy by day 100 (Notarangelo (2010) The Journal of Allergy and Clinical Immunology, 125(2, Suppl 2): S182-194). However, the possible risks of transplant (adverse effects or engraftment failure) have to be balanced with the benefit of available specific pharmaceutical treatments. These pharmaceutical treatments could also be essential to allow disease remission and making HSCT procedure less risky (Chan et al. (2020) Frontiers in Immunology, 11: 239) although a recent report revealed that the use of mTOR inhibitor post-HSCT may confer an advantage to residual host cells, promoting graft instability (Dimitrova et al. (2022) The Journal of Allergy and Clinical Immunology, 149(1): 410-421).

Accordingly, there is an ongoing need for novel and improved therapy approaches to treat Activated PI3kinase Delta Syndrome type 1 (APDS1) as well as other disorders related to mutations in the *PIK3CD* gene.

### SUMMARY OF THE INVENTION

The present invention addresses this need by providing, for the first time, an *ex vivo* gene editing approach for treating Activated PI3kinase Delta Syndrome type 1 (APDS1), in T-cells and/or in HSCs. Particularly, the present invention provides means and methods for genetically modifying HSCs or T-cells originating from a patient suffering from APDS1 involving gene-editing reagents, such as TALE-nucleases, that specifically target the endogenous *PIK3CD* locus. In particular, said gene-editing agents target at least a non-functional endogenous *PIK3CD* allele comprising at least one APDS1-associated mutation. As a result, engineered PIK3CD-edited HSCs and/or T-cells are provided which do not express a non-functional hyper-activated PI3Kδ protein. Preferably, the engineered PIK3CD-edited HSCs and/or T-cells provided herewith comprise an exogenous sequence comprising a nucleic acid sequence encoding a functional PI3Kδ protein, which is integrated in said HSCs' orT-cells' genome, at least within a non-functional endogenous *PIK3CD* allele and, optionally, also within a functional endogenous *PIK3CD* allele, thereby restoring the normal cellular phenotype by avoiding the expression of a non-functional PI3Kδ protein and enabling the expression of a functional PI3Kδ protein.

The present invention can be further summarized by the following items:
1. An engineered hematopoietic stem cell (HSC) or T-cell originating from a patient suffering from Activated PI3kinase Delta Syndrome type 1 ("APDS1"), comprising an exogenous polynucleotide sequence comprising the sequence of a functional *PIK3CD* gene ("PIK3CD sequence") or a portion thereof of at least 100 nucleotides, said exogenous polynucleotide sequence being integrated in the cell's genome in an endogenous *PIK3CD* allele comprising at least one mutation causing APDS1, thereby resulting in the presence, in said cell's genome, of a nucleic acid sequence encoding a functional PI3Kδ protein.
2. The engineered cell according to item 1, wherein said exogenous polynucleotide sequence comprises a PIK3CD sequence, or portion thereof, encoding the amino acid sequence of SEQ ID NO: 46 or a portion thereof of at least 35 amino acids.
3. The engineered cell according to claim 1 or 2, wherein said exogenous polynucleotide sequence comprises a PIK3CD sequence, or portion thereof, that has been codon optimized and, optionally, a polyA signal sequence placed at the 3' end of said PIK3CD sequence or portion thereof.
4. The engineered cell according to any one of items 1 to 3, wherein said exogenous polynucleotide sequence comprises a PIK3CD sequence, or portion thereof, comprising codon optimized sequences of exons 1 to 24 of *PIK3CD* or a portion thereof of at least 100 nucleotides.
5. The engineered cell according to any one of items 1 to 4, wherein said exogenous polynucleotide sequence integrated at any position within said endogenous *PIK3CD* allele in the cell's genome.
6. The engineered cell according to any one of items 1 to 5, wherein said exogenous polynucleotide sequence comprises a PIK3CD sequence, or portion thereof, comprising SEQ ID NO: 1 or a portion thereof of at least 100 nucleotides.
7. The engineered cell according to item 6, wherein said exogenous polynucleotide sequence is integrated within Exon 8 of said endogenous *PIK3CD* allele in the cell's genome.
8. The engineered cell according to any one of items 1 to 5, wherein said exogenous polynucleotide sequence comprises a PIK3CD sequence, or portion thereof, comprising SEQ ID NO: 2 or a portion thereof of at least 100 nucleotides, respectively.
9. The engineered cell according to item 8, wherein said exogenous polynucleotide sequence is integrated within Exon 17 of said endogenous *PIK3CD* allele in the cell's genome.
10. The engineered cell according to any one of items 1 to 5, wherein said exogenous polynucleotide sequence comprises a PIK3CD sequence, or portion thereof of at least 100 nucleotides, comprising a codon optimized sequence of Exon 24 of *PIK3CD.*
11. The engineered cell according to item 10, wherein said exogenous polynucleotide sequence is integrated within Exon 24 of said endogenous *PIK3CD* allele in the cell's genome.
12. The engineered cell according to any one of items 1 to 11, wherein said exogenous polynucleotide sequence further comprises a sequence encoding a surface marker such as LNGFR, EGFRt, appropriate for purification and/or detection of said engineered cell.
13. The engineered cell according to any one of items 1 to 12, further comprising an exogenous polynucleotide sequence comprising the sequence of a functional *PIK3CD* gene ("PIK3CD sequence") or a portion thereof of at least 100 nucleotides, said exogenous polynucleotide sequence being integrated in the cell's genome in an endogenous *PIK3CD* allele comprising no APDS1-associated mutation.
14. The engineered cell according to item 13, wherein each of the two exogenous polynucleotide sequences further comprises a sequence encoding a surface marker, wherein the surface markers are different.
15. A population of cells comprising engineered cells according to any one of items 1 to 14.
16. The population of cells according to item 15, comprising at least 1%, at least 5%, at least 6%, at least 7%, 8%, at least 9%, at least 10%, at least 15%, at least 20%, at least 25%, at least 30%, at least 35%, or at least 40% of said engineered cells.
17. A TALE-Nuclease heterodimer targeting a polynucleotide sequence comprised within the *PIK3CD* gene, preferably targeting the polynucleotide sequence of SEQ ID NO: 3 within Exon 8 of *PIK3CD,* or SEQ ID NO: 6 within Exon 17 of *PIK3CD,* or SEQ ID NO: 9 within Exon 24 of *PIK3CD* gene.
18. The TALE-Nuclease heterodimer according to item 17, comprising a pair of monomers comprising repeat sequences comprising repeat-variable diresidues (RVDs) selected from the group consisting of:
   a) a first monomer comprising the RVDs NN-NI-NI-HD-NN-HD-HD-NN-NI-HD-NN-NI-NN-HD-NN-NG# and a second monomer comprising the RVDs HD-HD-HD-NI-HD-HD-NG-NI-NG-HD-HD-HD-NI-NN-NN-NG#, wherein the heterodimer targets Exon 8 of *PIK3CD* gene;
   b) a first monomer comprising the RVDs NN-NI-NG-NN-HD-NI-HD-NG-NG-NN-NG-NN-HD-NI-NG-NG# and a second monomer comprising the RVDs NN-NN-NN-NI-NN-NI-NN-NN-NN-HD-HD-NG-HD-NG-NI-NG#, wherein the heterodimer targets Exon 17 of *PIK3CD* gene; and
   c) a first monomer comprising the RVDs NN-NN-HD-NI-HD-NG-NN-NN-NN-NN-NI-NI-NI-NI-HD-NG# and a second monomer comprising the RVDs NN-NG-NG-NI-NI-NI-HD-NG-NG-HD-NI-HD-NG-HD-NN-NG#, wherein the heterodimer targets Exon 24 of *PIK3CD* gene.
19. A TALE-Nuclease heterodimer according to item 17 or 18, comprising repeat sequences comprising RVDs, in particular at position 12 and/or position 13, which are X*, wherein X represents one amino acid residue selected from the group consisting of A, G, V, L, I, M, S, T, C, P, D, E, F, Y, W, Q, N, H, R and K, preferably N, Q, T or H, and * represents a deletion in position 13 of the RVD; or comprising repeat sequences comprising RVDs, in particular at position 12 and position 13, which are NA, HA, or RG.
20. The TALE-Nuclease heterodimer according to item 17 or 18, wherein said TALE-Nuclease heterodimer targets the polynucleotide sequence of SEQ ID NO: 9 within Exon 24 of *PIK3CD* gene, and comprises a pair of monomers comprising repeat sequences comprising RVDs, in particular at position 12 and/or position 13, which are N*or H*, wherein * represents a deletion in position 13 of the RVD.
21. The TALE-Nuclease heterodimer according to any one of items 17 to 20, comprising:
   a) a first monomer comprising the amino acid sequence of SEQ ID NO: 40 or a variant thereof comprising an amino acid sequence having at least 80% sequence identity with SEQ ID NO: 40, and a second monomer comprising the amino acid sequence of SEQ ID NO: 41 or a variant thereof comprising an amino acid sequence having at least 80% sequence identity with SEQ ID NO: 41, wherein the heterodimer targets the sequence of SEQ ID NO: 3;
   b) a first monomer comprising the amino acid sequence of SEQ ID NO: 42 or a variant thereof comprising an amino acid sequence having at least 80% sequence identity with SEQ ID NO: 42, and a second monomer comprising the amino acid sequence of SEQ ID NO: 43 or a variant thereof comprising an amino acid sequence having at least 80% sequence identity with SEQ ID NO: 43, wherein the heterodimer targets the sequence of SEQ ID NO: 6; or
   c) a first monomer comprising the amino acid sequence of SEQ ID NO: 44 or a variant thereof comprising an amino acid sequence having at least 80% sequence identity with SEQ ID NO: 44, and a second monomer comprising the amino acid sequence of SEQ ID NO: 45 or a variant thereof comprising an amino acid sequence having at least 80% sequence identity with SEQ ID NO: 45, wherein the heterodimer targets the sequence of SEQ ID NO: 9.
22. The TALE-Nuclease heterodimer according to item 17, wherein said TALE-Nuclease heterodimer targets the polynucleotide sequence of SEQ ID NO: 3 within Exon 8 of *PIK3CD* gene.
23. The TALE-Nuclease heterodimer according to item 22, comprising a first monomer comprising the amino acid sequence of SEQ ID NO: 40 and a second monomer comprising the amino acid sequence of SEQ ID NO: 41.
24. An isolated nucleic acid or vector encoding a TALE-Nuclease heterodimer according to any one of items 17 to 23.
25. The isolated nucleic acid according to item 24, which is a mRNA.
26. An isolated nucleic acid or a vector comprising an exogenous polynucleotide sequence as defined in any one of items 1 to 12, wherein said exogenous sequence is placed between a left homologous region at its 5' end and a right homologous region at its 3' end, homologous regions being relative to the targeted portion of the *PIK3CD* gene.
27. A population of cells comprising HSCs or T-cells comprising (i) a TALE-Nuclease heterodimer according to any one of items 17 to 23 or an isolated nucleic acid or vector encoding said TALE-Nuclease heterodimer, and (ii) an exogenous polynucleotide sequence as defined in any one of items 1 to 12.
28. A pharmaceutical composition comprising an engineered cell according to any one of items 1 to 14, or a population of cells according to any one of items 15 to 16, and a pharmaceutically acceptable excipient and/or carrier.
29. An engineered cell according to any one of items 1 to 14, a population of cells according to any one of items 15 to 16, or a pharmaceutical composition according to item 28, for use in the treatment of Activated PI3kinase Delta Syndrome type 1.
30. An engineered HSC according to any one of items 1 to 14, a population of cells according to any one of items 15 to 16, or a pharmaceutical composition according to item 28, for use in haematopoietic stem cell transplantation.
31. A kit comprising:
   (i) at least one isolated nucleic acid or vector comprising an exogenous polynucleotide sequence according to item 26, and
   (ii) at least one isolated nucleic acid or vector encoding a TALE-nuclease heterodimer according to any one of items 24 to 25.
32. The kit according to item 31 comprising:
   (i) an isolated nucleic acid or vector comprising the nucleic acid sequence SEQ ID NO: 1 and, optionally, a nucleic acid sequence encoding a surface marker; and at least one isolated nucleic acid or vector comprising a nucleic acid sequence encoding the amino acid sequences of SEQ ID NO: 40 and/or SEQ ID NO: 41;
   (ii) an isolated nucleic acid or vector comprising the nucleic acid sequence SEQ ID NO: 2 and, optionally, a nucleic acid sequence encoding a surface marker; and at least one isolated nucleic acid or vector comprising a nucleic acid sequence encoding the amino acid sequences of SEQ ID NO: 42 and/or SEQ ID NO: 43; and/or
   (iii) an isolated nucleic acid or vector comprising a codon optimized sequence of Exon 24 of *PIK3CD* and, optionally, a nucleic acid sequence encoding a surface marker; and at least one isolated nucleic acid or vector comprising a nucleic acid sequence encoding the amino acid sequences of SEQ ID NO: 44 and/or SEQ ID NO: 45.
33. *Ex vivo* use of the TALE-Nuclease heterodimer according to any one of items 17 to 23, or an isolated nucleic acid or vector encoding said TALE-Nuclease heterodimer, or the kit according to item 31 or 32, for integrating a functional *PIK3CD* sequence in any one of Exons 1 to 24 of an endogenous *PIK3CD* locus of an HSC or T-cell.
34. The *ex vivo* use of item 33, wherein said functional PIK3CD sequence is integrated at least in a non-functional endogenous *PIK3CD* allele and, optionally, also in a functional *PIK3CD* allele.
35. A method of engineering HSCs or T-cells having a non-functional endogenous *PIK3CD* allele comprising at least one APDS1-associated mutation in said cells, said method comprising introducing into said cells:
   (i) at least one sequence specific reagent inducing DNA cleavage that is capable of specifically targeting and cleaving a sequence within an endogenous *PIK3CD* locus, in at least a non-functional *PIK3CD* endogenous allele, in said cells;
   (ii) at least one isolated nucleic acid or vector comprising an exogenous sequence as defined in any one of items 1 to 12, wherein said exogenous sequence is placed between a left homologous region at its 5' end and a right homologous region at its 3' end, homologous regions being relative to the targeted portion of the *PIK3CD* gene;
   whereby engineered PIK3CD-edited HSCs or T-cells are obtained, which comprise a non-functional *PIK3CD* allele that has been disrupted and comprise a nucleic acid sequence encoding a functional PI3Kδ protein.
36. The method according to item 35, wherein said exogenous sequence in (ii) further comprises a nucleic acid sequence encoding a surface marker.
37. The method according to item 35 or 36, wherein said sequence specific reagent inducing DNA cleavage is a TALE-nuclease, a meganuclease, a MegaTal nuclease, a zing-finger nuclease (ZFN), or a RNA guided endonuclease.
38. The method according to item 37, wherein said sequence specific reagent inducing DNA cleavage is the TALE-nuclease heterodimer according to any one of items 17 to 23.
39. The method according to item 35 or 36, wherein said sequence specific reagent inducing DNA cleavage is a TALE-nuclease heterodimer comprising a first monomer comprising the amino acid sequence of SEQ ID NO: 40 and a second monomer comprising the amino acid sequence of SEQ ID NO: 41.
40. The method according to any one of items 35 to 39, wherein:
   a) said sequence specific reagent of (i) comprises a nucleic acid sequence encoding the amino acid sequences of SEQ ID NO: 40 and SEQ ID NO: 41, and said nucleic acid or vector of (ii) comprises an exogenous sequence comprising the nucleic acid sequence SEQ ID NO: 1 and optionally a nucleic acid sequence encoding a surface marker;
   b) said sequence specific reagent of (i) comprises a nucleic acid sequence encoding the amino acid sequences of SEQ ID NO: 42 and SEQ ID NO: 43, and said nucleic acid or vector of (ii) comprises an exogenous sequence comprising the nucleic acid sequence SEQ ID NO: 2 and optionally a nucleic acid sequence encoding a surface marker; or
   c) said sequence specific reagent of (i) comprises a nucleic acid sequence encoding the amino acid sequences of SEQ ID NO: 44 and SEQ ID NO: 45, and said nucleic acid or vector of (ii) comprises an exogenous sequence comprising a codon optimized sequence of Exon 24 of *PIK3CD* and optionally a nucleic acid sequence encoding a surface marker.
41. The method according to any one of items 35 to 40, wherein said HSCs or T-cells to be engineered are isolated from a tissue sample from a human patient suffering from Activated PI3kinase Delta Syndrome type 1.
42. The method according to item 41, wherein the tissue sample is a peripheral blood sample.
43. The method according to any one of items 35 to 42, wherein said sequence specific reagent in (i) is introduced into the cells as mRNA by electroporation.
44. The method according to any one of items 35 to 43, wherein said vector in (ii) is a non-integrative viral vector such as an adeno-associated virus (AAV) or an Integrationdeficient Lentiviral Particle (IDLV).
45. The method according to item 44, wherein said vector is an AAV vector, such as an AAV6 vector.
46. The method according to any one of items 35 to 45, wherein said at least one isolated nucleic acid or vector of (ii) comprises two of said exogenous polynucleotide sequences, wherein each of the two exogenous polynucleotide sequences further comprises a sequence encoding a surface marker and wherein the surface markers are different.
47. A method of purifying engineered PIK3CD-edited HSCs or T-cells originating from a patient suffering from APDS1, comprising:
   (i) providing a population of cells comprising the engineered HSCs or T-cells according to item 12 or the population of cells obtained by the method according to item 36;
   (ii) binding the engineered HSCs or T-cells with a binding agent specific for said surface marker, wherein said binding agent is fixed to a support; and
   (iii) eluting the engineered HSCs or T-cells from the support;
   wherein the eluted product is enriched in PIK3CD-edited HSCs or T-cells.
48. A method of purifying engineered PIK3CD-edited HSCs or T-cells originating from a patient suffering from APDS1, comprising:
   (i) providing a population of cells comprising the engineered HSCs or T-cells according to item 14 or the population of cells obtained by the method according to item 46;
   (ii) binding said engineered HSCs or T-cells with a binding agent specific for a first surface marker and with a binding agent specific for a second surface marker, wherein said binding agents are fixed to a support and wherein said first and second surface markers are different;
   (iii) eluting the engineered HSCs or T-cells from the support;
   wherein the eluted product is enriched in PIK3CD-edited HSCs or T-cells comprising
   (a) an exogenous polynucleotide sequence comprising the sequence of a functional *PIK3CD* gene (or a portion thereof of at least 100 nucleotides) and a sequence encoding a first surface marker, said sequences being integrated in the cell's genome in an endogenous *PIK3CD* allele comprising at least one APDS1-associated mutation; and
   (b) an exogenous polynucleotide sequence comprising the sequence of a functional *PIK3CD* gene or a portion thereof of at least 100 nucleotides and a sequence encoding a second surface marker, said sequences being integrated in the cell's genome in an endogenous *PIK3CD* allele comprising no APDS1-associated mutation.
49. An engineered hematopoietic stem cell (HSC) or T-cell originating from a patient suffering from APDS1, wherein the endogenous *PIK3CD* allele comprising at least one APDS1 associated mutation has been cleaved by a sequence specific reagent, thereby resulting in the expression of only a functional *PIK3CD* gene in said engineered cell.
50. A population of cells comprising HSCs or T-cells according to item 49.
51. A pharmaceutical composition comprising an engineered cell according to item 49, or a population of cells according to item 50, and a pharmaceutically acceptable excipient and/or carrier.
52. An engineered cell according to item 49, a population of cells according to item 50, or a pharmaceutical composition according to item 51, for use in the treatment of Activated PI3kinase Delta Syndrome type 1.
53. An engineered HSC according to item 49, a population of cells according to item 50, or a pharmaceutical composition according to item 51, for use in haematopoietic stem cell transplantation.
54. A method of engineering HSCs or T-cells comprising introducing into HSCs or T-cells having an endogenous *PIK3CD* gene comprising at least one APDS1-associated mutation, at least one sequence specific reagent inducing DNA cleavage that is capable of specifically targeting and cleaving a sequence within a non-functional endogenous *PIK3CD* gene in said cells;
   whereby engineered PIK3CD-edited HSCs or T-cells are obtained, which have a non-functional *PIK3CD* gene that has been disrupted and comprise an endogenous nucleic acid sequence encoding a functional PI3Kδ protein.
55. The method according to item 54, wherein said sequence specific reagent is a TALE-nuclease, a meganuclease, a megaTal nuclease, a zing-finger nuclease (ZFN), or a RNA guided endonuclease.
56. *Ex vivo* use of a sequence specific reagent inducing DNA cleavage that is capable of specifically targeting and cleaving a sequence within the endogenous non-functional *PIK3CD* allele comprising at least one APDS1-associated mutation in an HSC or T-cell, for disrupting said endogenous non-functional *PIK3CD* in said HSC or T-cell.

### BRIEF DESCRIPTION OF THE DRAWINGS

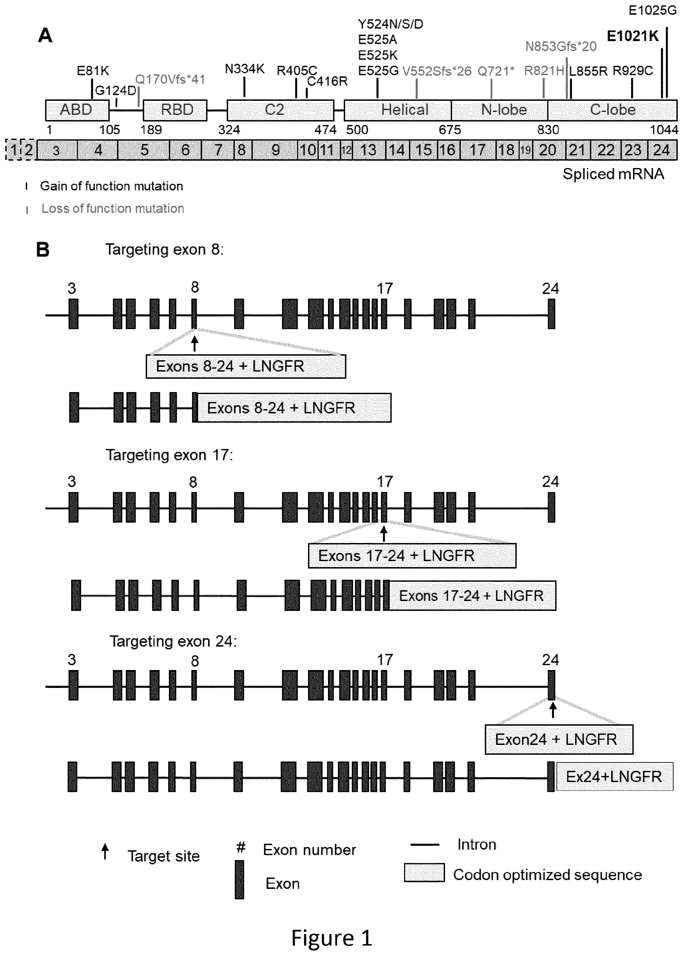

**Figure 1:** (A) p110delta protein and associated exons of corresponding *PIK3CD* gene. The different functional domains of the protein are depicted. Gain of function and loss of function mutations are indicated in black or in grey, respectively. (B). Strategies for APDS1 phenotype correction. Target sites in exons 8, 17 and 24 of *PIK3CD* gene are indicated by a black arrow. Exons and introns are in black vertical or horizontal bars, respectively. The resulting PIK3CD-edited unspliced mRNA is depicted below the genomic DNA.
   References for the indicated mutations in *PIK3CD* gene are as follows:
   E81K: Takeda et al. J Allergy Clin Immunol. (2017) 140(4): 1152-1156; Heurtier et a/. (2017) Haematologica, 102(7): e278-e281;
   G124D: Takeda *et al.* (2017) supra; Heurtier *et al.* (2017) supra;
   Q170Vfs*41 : Swan et al. (2019) Haematologica 104:e483-e486;
   N334K: Lucas *et al.* (2014) supra;
   R405C: Rae et al. (2017) Clin Immunol. 181: 29-31;
   C416R: Crank et al. (2014) J Clin Immunol. 34:272-6;
   Y524N: Luo et al. (2018) Clin Immunol. 197: 60-7;
   Y524S: Thauland et al. (2019) Front Immunol. 10: 753;
   Y524D: Tessarin et al. (2020) J Clin Med. 9: 3335;
   E525K: Lucas *et al.* (2014) supra;
   E525A: Tsujita et a/ (2016) J Allergy Clin Immunol, 138(6), 1672-1680;
   E525G: Marzollo et al (2021) Front Pediatric 9:703056;
   V552Sfs*26: Sogkas et al. (2018) J Allergy Clin Immunol. 142(5):1650-1653;
   Q721*: Sharfe et a/ (2018) J Allergy Clin Immunol. 142618-629;
   R821H: Rodriguez et al (2019) J Exp Med. 216(12): 2800-2818;
   N853Gfs*20 : Cohen et al. (2019) J Allergy Clin Immunol. 143(2): 797-799.e2;
   L855R: Carey et al (2020) Pediatr Res. 88(5): 761-768.
   R929C: Wentink et al. (2017) Clin Immunol. 176: 77-86;
   E1021K : Angulo et al. (2013) Science, 342(6160): 866-871 ; Lucas *et al.* (2014), supra;
   E1025G : Dulau et al. (2016) J Allergy Clin Immunol.139: 1032-5.

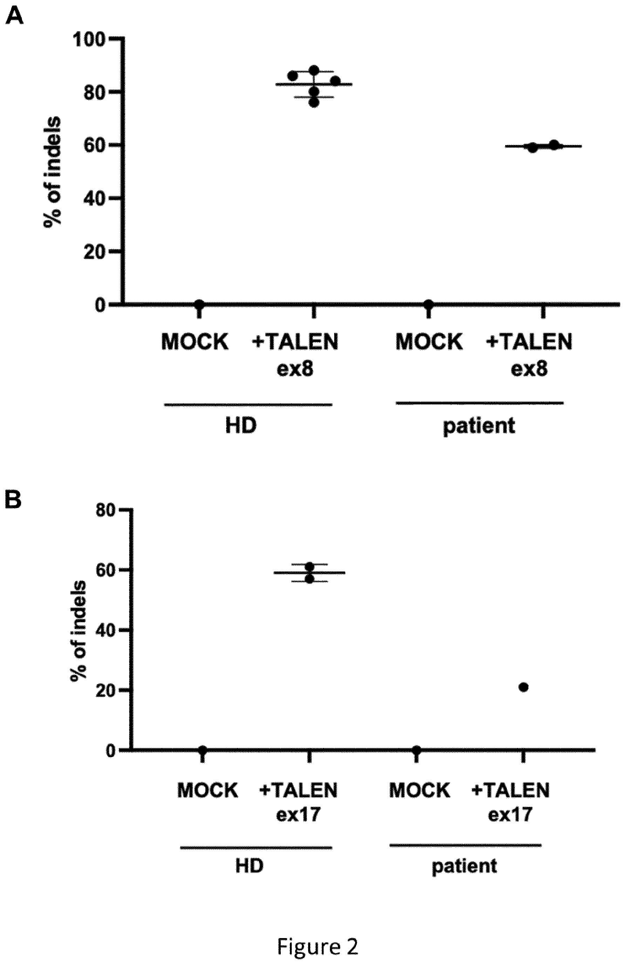

**Figure 2****:** Scatter plot of indel frequencies ("% of indels") measured by ddPCR of Healthy Donor ("HD") or APDS1 patient ("patient") T-cells electroporated or not ("MOCK") with mRNA encoding the TALE-nuclease targeting either exon 8 ("+TALENex8") (A) or exon 17 ("+TALENex17") of PIK3CD (**B**).

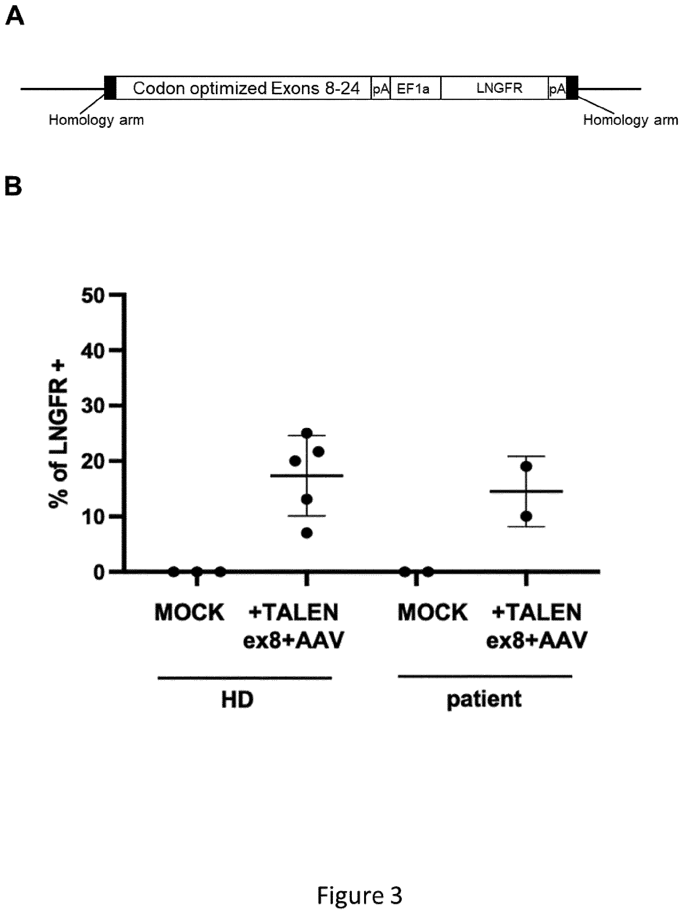

**Figure 3**: (**A**) Schematic representation of codon optimized PIK3CD exons 8 to 24 transcript expression cassette co-expressed with LNGFR via an EF1a promotor. (**B**) Scatter plot of LNGFR-positive cells (%) after AAV transduction with ("+TALENex8+AAV") or without ("MOCK") TALE-nuclease mRNA electroporation in Healthy Donor ("HD") or APDS1 patient ("patient") T-cells.

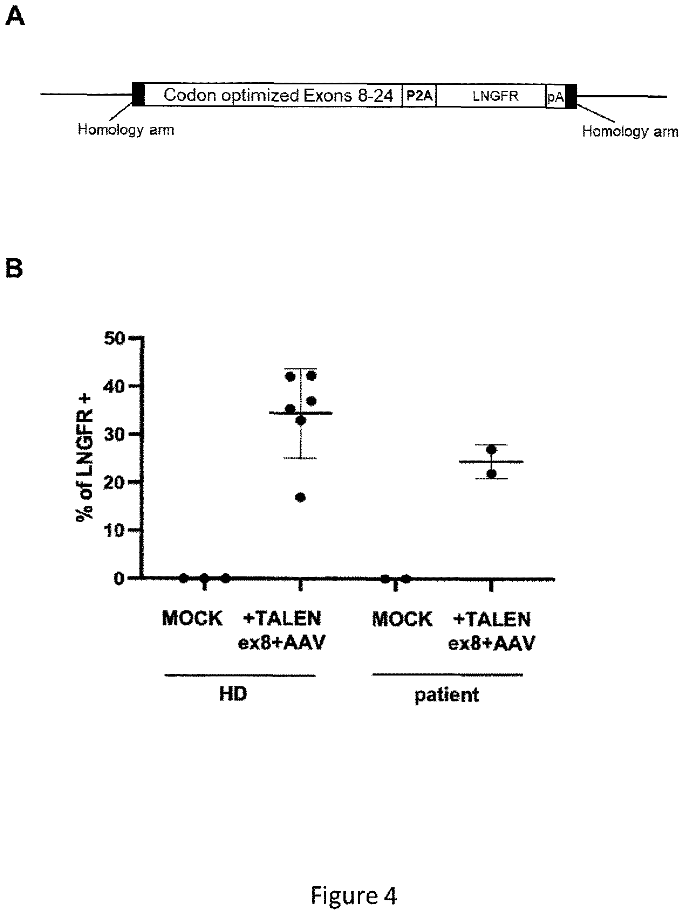

**Figure 4:** (A) Schematic representation of codon optimized PIK3CD exons 8 to 24 transcript expression cassette co-expressed with LNGFR via P2A. (B) Scatter plot of LNGFR-positive cells (%) after AAV transduction with ("+TALENex8+AAV") or without ("MOCK") TALE-nuclease mRNAs electroporation in Healthy Donor ("HD") or APDS1 patient ("patient") T-cells.

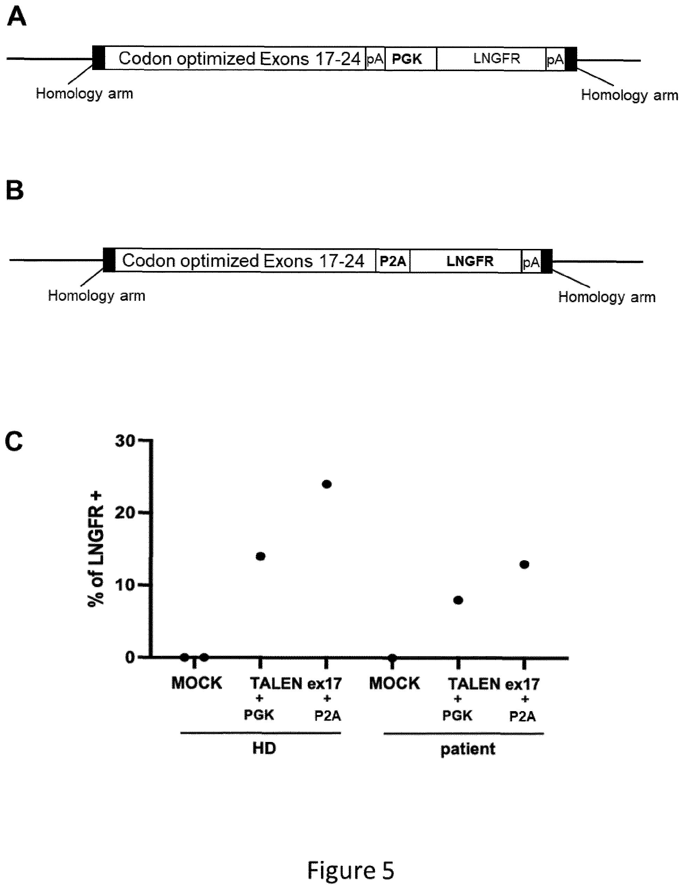

**Figure 5****:** (**A**) Schematic representation of codon optimized PIK3CD exons 17 to 24 transcript expression cassette co-expressed with LNGFR via PGK promoter. (**B**) Schematic representation of codon optimized PIK3CD exons 17 to 24 transcript expression cassette co-expressed with LNGFR via P2A. (**C**) Scatter plot of LNGFR-positive cells (%) after electroporation with TALEN targeting exon 17 and after transduction with exon 17-targeting AAV vector comprising LNGFR expression under PGK promoter ("PGK") or via a P2A link ("P2A") in Healthy Donors ("HD") or APDS1 patients ("patient") T-cells.

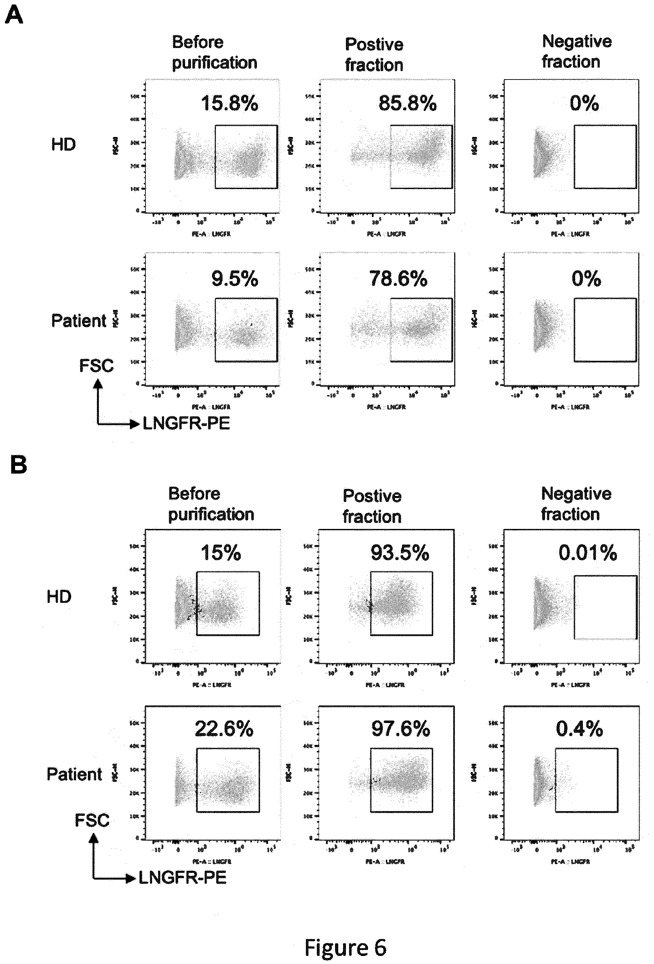

**Figure 6**: Flow cytometry results before and after LNGFR purification of Healthy Donors ("HD") or APDS1 patients ("patient") T-cells obtained after treatment with TALE-nuclease and AAV vector containing EF1a-LNGFR (A) or P2A-LNGFR (**B**).

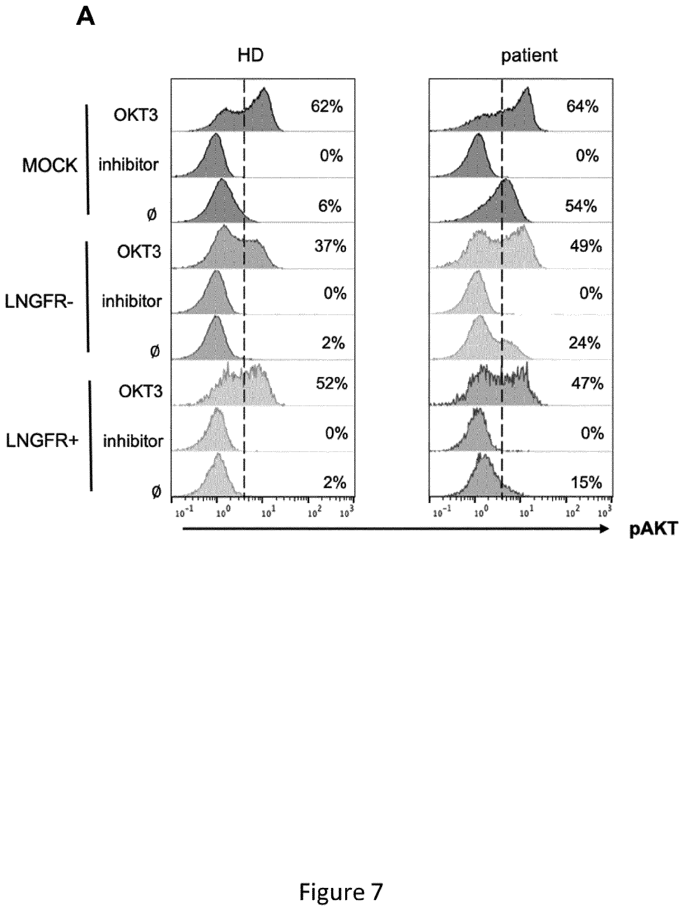

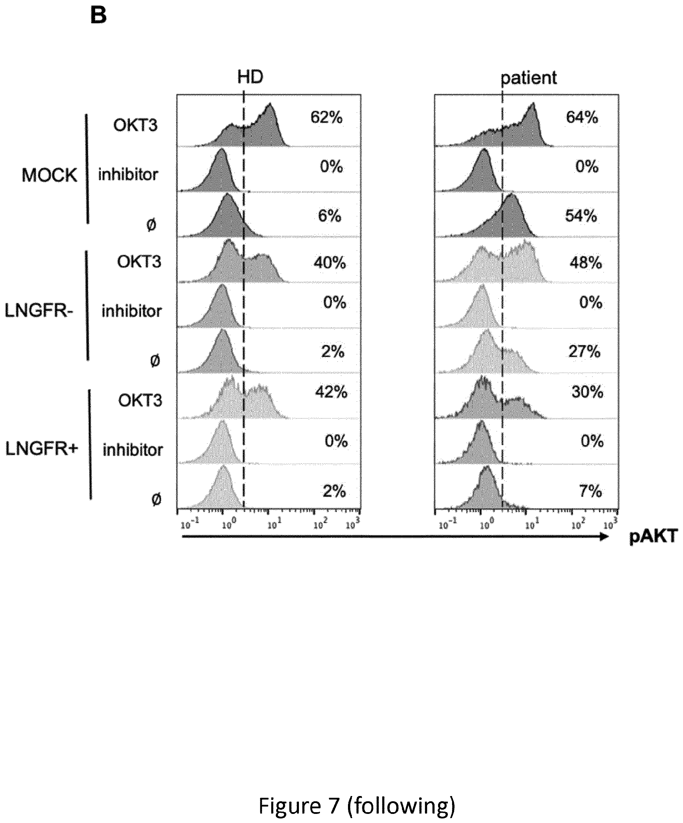

**Figure 7****:** Cytometry analysis revealing pAKT level in untreated ("MOCK") or in LNGFR-positive ("LNGFR+") or LNGFR-negative ("LNGFR-") fraction obtained after treatment with TALEN targeting exon 8 and AAV vector containing exons8-24-EF1a-LNGFR cassette (**A**) or exons8-24-P2A-LNGFR cassette (**B**). The pAKT levels were estimated without any stimulation, after OKT3 activation or in presence of a PI3K inhibitor.

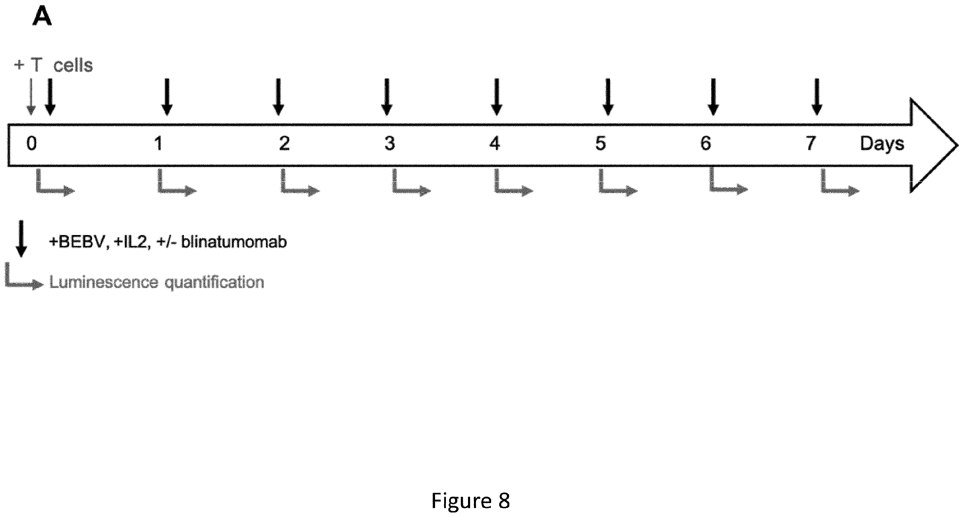

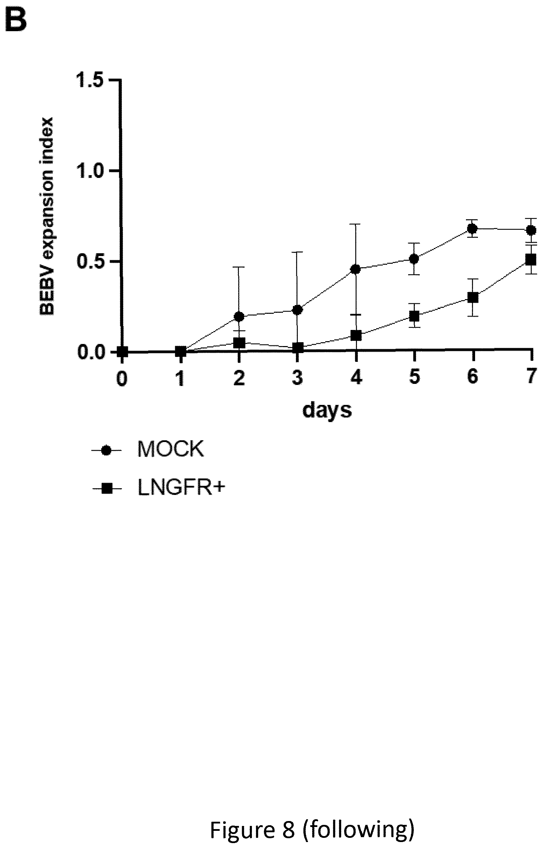

**Figure 8****:** (A) Schematic representation of cytotoxic assay. (B) B-EBV expansion index at different days of co-culture of B-EBV cells with either non-engineered APDS1 patient T-cells ("MOCK") or purified engineered APDS1 patient T-cells ("LNGFR+"). Mean and standard deviation of two independent experiments obtained with 2 independent engineered APDS1 patient T-cells.

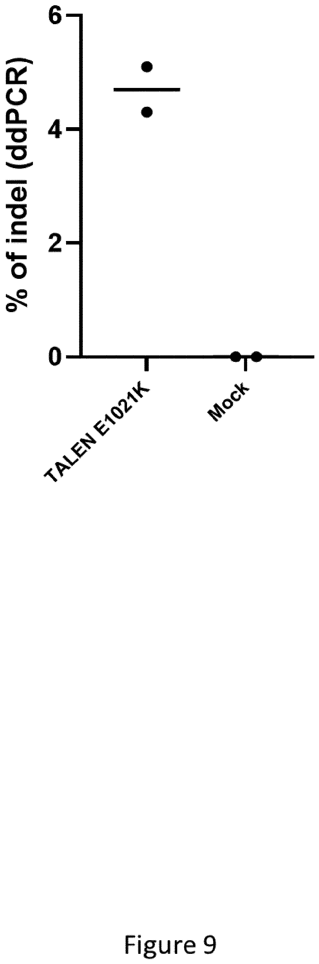

**Figure 9**: Scatter plot of indel frequencies (% of indels) measured by ddPCR of APDS1 patient T-cells electroporated with mRNAs encoding a TALE-nuclease specific for the E1021 K mutation.

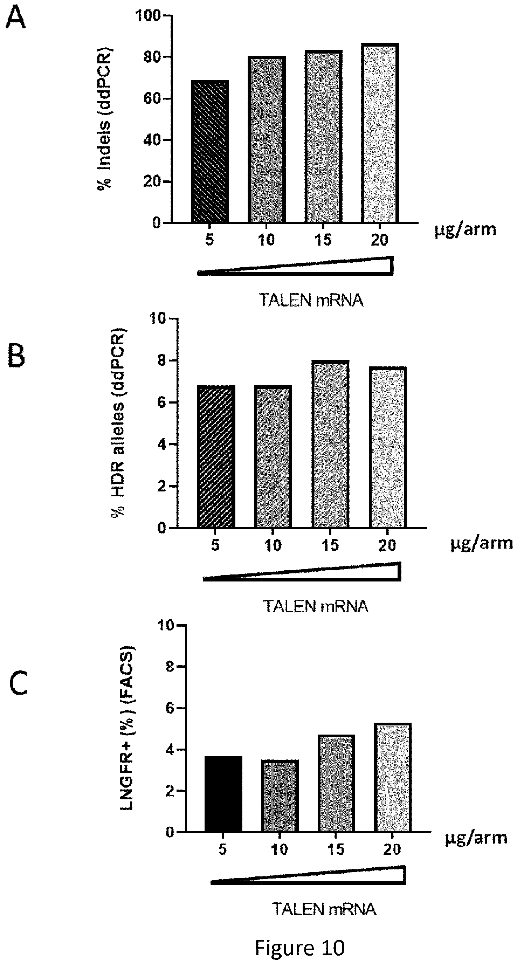

**Figure 10****:** (**A**) Indel frequencies (% of indels) measured by ddPCR of healthy donors' HSCs electroporated with increasing amount of mRNAs encoding a TALE-nuclease targeting PIK3CD exon 8. HDR frequency measured by ddPCR (**B**); or by percentage of LNGFR positive cells obtained (**C**), in healthy donors' HSCs electroporated with the indicated amount of mRNAs encoding a TALE-nuclease targeting exon 8 and transduced with 5e4 MOI dose of AAV vector delivering the DNA homology donor cassette EF1a-LNGFR.

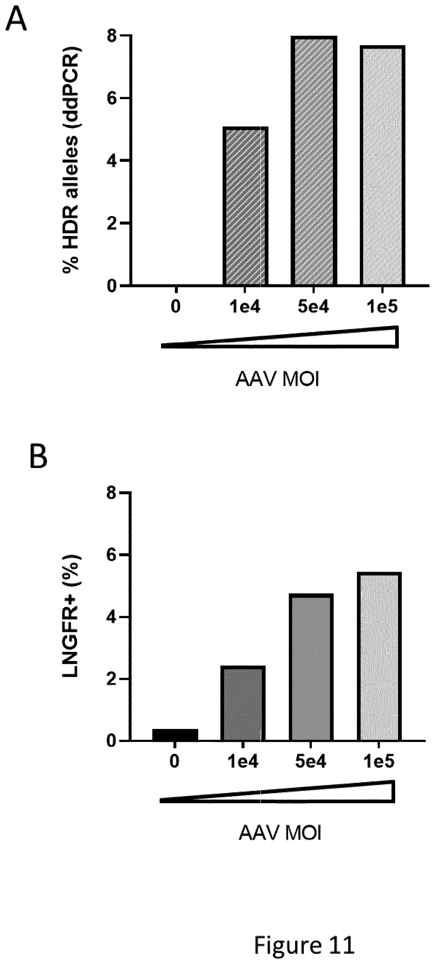

**Figure 11****:** HDR frequency measured either by ddPCR (A) or by percentage of LNGFR-positive cells obtained (**B**), in healthy donors' HSCs electroporated with mRNAs encoding a TALE-nuclease targeting PIK3CD exon 8 and transduced with AAV vector delivering the DNA homology donor cassette EF1a-LNGFR.

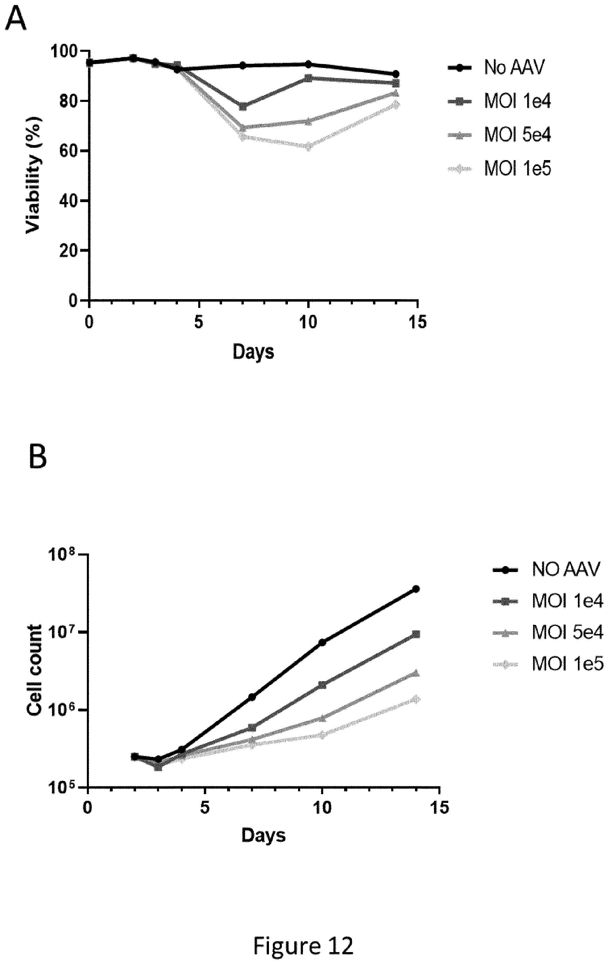

**Figure 12****:** Viability (**A**) or proliferation (**B**) depending on the dose of AAV transduced

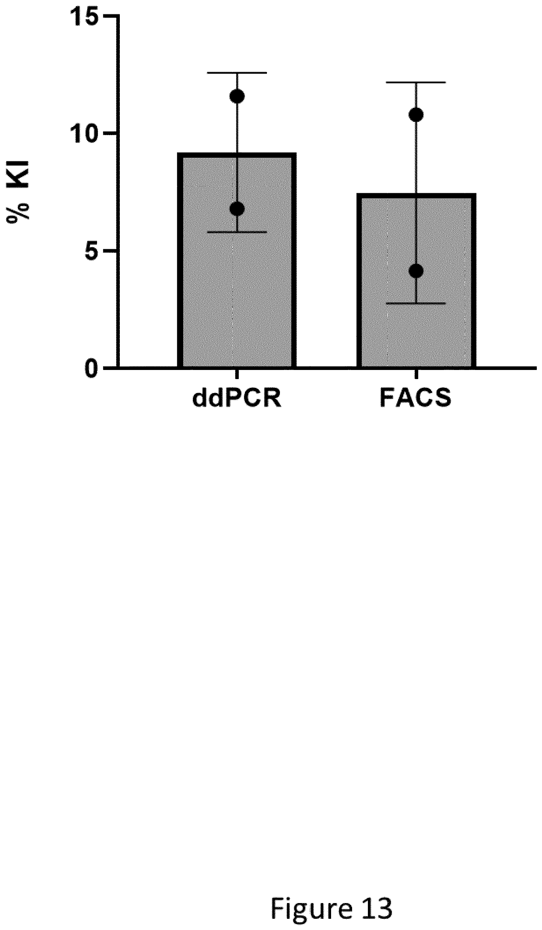

**Figure 13****:** Targeted integration ("% KI") measured either by ddPCR ("ddPCR") or by percentage of LNGFR-positive cells obtained ("FACS"), in healthy donors' HSCs electroporated with optimal dose of mRNAs encoding a TALE-nuclease targeting PIK3CD exon 8 and transduced with optimal dose response of AAV vector delivering the DNA homology donor cassette EF1a-LNGFR.

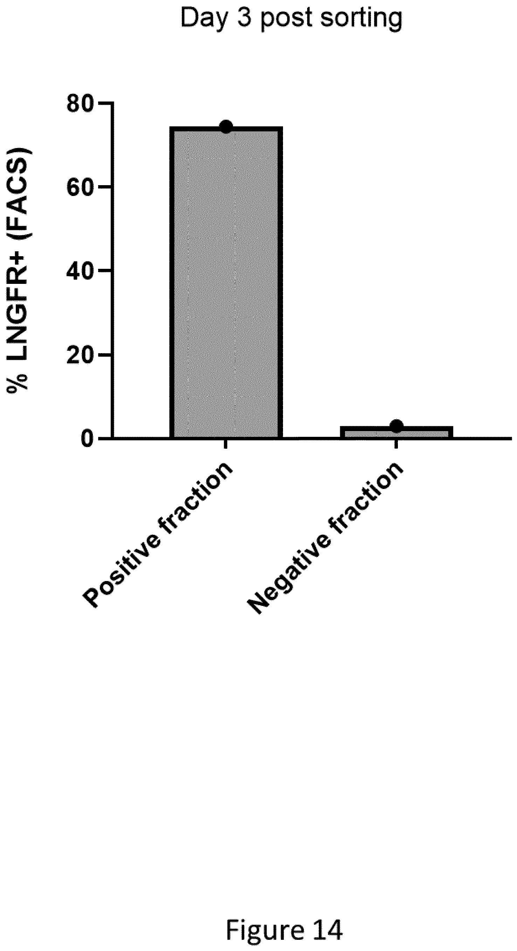

**Figure 14****:** Percentage of LNGFR-positive HSCs obtained in the positive and negative fractions three days post sorting.

### DETAILED DESCRIPTION OF THE INVENTION

Unless specifically defined herein, all technical and scientific terms used herein have the same meaning as commonly understood by a skilled artisan in the fields of gene therapy, biochemistry, genetics, and molecular biology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, genetics, transgenic biology, microbiology, recombinant DNA, and immunology, which are within the skill of the art. Such techniques are explained fully in the literature. See, for example, Current Protocols in Molecular Biology [Frederick M. AUSUBEL (2000) Wiley and son Inc, Library of Congress, USA; Molecular Cloning: A Laboratory Manual, Third Edition] [Sambrook et al (2001) Cold Spring Harbor, New York: Cold Spring Harbor Laboratory Press]; Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); and Manipulating the Mouse Embryo, (Cold Spring Harbor Laboratory Press, Cold Spring Harbor, N.Y., 1986).

### Cells and cell populations of the present invention

As noted above, the present invention is an improved gene therapy approach to treat Activated PI3kinase Delta Syndrome type 1 (APDS1) related to *PIK3CD* gene, allowing the correction of the phenotype related to PIK3CD deficiency in HSCs and T-cells. Human *PIK3CD* gene is described at NCBI Reference NG_023434.1. It comprises 24 exons.

As APDS1 is generally caused by one or more dominant mutations in the *PIK3CD* gene, the present invention is drawn to an engineered HSC or T-cell originating from a patient suffering from APDS1, wherein the endogenous *PIK3CD* gene comprising at least one mutation causing APDS1 has been cleaved by a sequence specific reagent, thereby resulting in the absence of expression of the hyper-activated non-functional PI3Kδ protein.

Particularly, the present invention provides means and methods for genetically modifying HSCs and/or T-cells involving gene-editing reagents, such as TALE-nucleases, that target the PIK3CD locus, in particular at least a PIK3CD allele comprising at least one mutation causing APDS1. As a result, engineered PIK3CD-edited HSCs and/or T-cells are provided which do not express a non-functional hyper-activated PI3Kδ protein.

In one aspect, the engineered PIK3CD-edited HSCs and/orT-cells provided herewith comprise an exogenous sequence comprising a nucleic acid sequence encoding a functional PI3Kδ protein, which is integrated in said HSCs' or T-cells' genome within an endogenous PIK3CD locus, in particular at least in a PIK3CD allele comprising at least one APDS1-associated mutation, thereby restoring the normal cellular phenotype by enabling the expression of a functional PI3Kδ protein.

Given that Activated PI3kinase Delta Syndrome type 1 ("APDS1") is caused by one or more mutations which can be spread along the entire *PIK3CD* gene which encodes the entire PI3Kδ protein, the present invention particularly aims at integrating in the HSC's or T-cell's genome, anywhere in the defective endogenous *PIK3CD* locus, an exogenous polynucleotide sequence comprising the sequence of a functional *PIK3CD* gene ("PIK3CD sequence") or a portion thereof of at least 100 nucleotides, thereby resulting in the expression of a functional PI3Kδ polypeptide.

Thus, in a general aspect, the present invention is drawn to an engineered HSC or T-cell originating from a patient suffering from APDS1, comprising an exogenous polynucleotide sequence comprising the sequence of a functional *PIK3CD* gene ("PIK3CD sequence") or a portion thereof of at least 100 nucleotides, said exogenous polynucleotide sequence being integrated in the cell's genome in an endogenous *PIK3CD* locus, in at least a *PIK3CD* allele comprising at least one APDS1-associated mutation, thereby resulting in the presence, in said cell's genome, of a nucleic acid sequence encoding a functional PI3Kδ protein. As a result of the integration of said exogenous sequence within an endogenous *PIK3CD* allele comprising at least one APDS1-associated mutation, said endogenous non-functional *PIK3CD* gene is disrupted and does not allow the expression of a non-functional hyper-activated PI3Kδ protein.

In another general aspect, the engineered cells described herewith comprise an exogenous polynucleotide sequence comprising a PIK3CD sequence, or portion thereof, encoding the amino acid sequence of SEQ ID NO: 46 or a portion thereof of at least 35 amino acids.

With "functional PI3Kδ protein", "functional PI3Kδ catalytic subunit p110δ", "functional p110δ", it is meant the PI3Kδ protein isoform 1 (NCBI reference NP_005017.3) of SEQ ID NO: 46. A functional PI3Kδ protein is able to associate with the other subunits of PI3K protein to allow conversion of phosphatidylinositol 4,5-bisphosphate into phosphatidylinositol 3,4,5-trisphosphate (PIP3). A possible read-out of the activity of the PI3Kd protein is to determine the downstream activation of AKT (also called "protein kinase B") by determining the level of phosphorylation of AKT by standard methods including the one described in Donahue et al. (2007) (Methods in Enzymology,Academic Press, 434:131-154).

With "non-functional endogenous *PIK3CD* allele", it is meant an allele of an endogenous *PIK3CD* gene, which contains one or more mutations associated with a disease state such as Activated PI3kinase Delta Syndrome type 1 (APDS1), refractory immune thrombocytopenia, inflammatory bowel disease or susceptibility to infection. To date, at least 15 PIK3CD mutations have been identified in APDS1 patients and 5 PIK3CD mutations have been identified in non-APDS1 patients such as patients suffering from refractory immune thrombocytopenia, inflammatory bowel disease or susceptibility to infection (see Figure 1A and references cited in the legend of Figure 1A). As defined herewith a non-functional *PIK3CD* gene encodes a non-functional PI3Kδ protein. A non-functional PI3Kδ protein designates a protein whose amino acid sequence comprises APDS1-related mutations which are generally gain of function mutations, resulting in a non-functional hyper-activated PI3Kδ protein. A non-functional PI3Kδ protein also includes a protein whose amino acid sequence comprises non-APDS1-related mutations which are loss of function mutations, resulting in a shortened or less active PI3Kδ protein, as in the case of mutations in the *PIK3CD* gene which are not related to APDS1 but to other disorders like refractory immune thrombocytopenia, inflammatory bowel disease and susceptibility to infection. In general, in the case of APDS1-related mutations in the *PIK3CD* gene, a non-functional *PIK3CD* allele encodes a non-functional PI3Kδ protein that is hyper-activated. A non-functional endogenous *PIK3CD* allele can comprise at least one mutation associated with APDS1. The mutations in the *PIK3CD* locus associated with APDS1 include the mutations E81K, G124D, N334K, R405C, C416R, Y524N, Y524S, Y524D, E525A, E525K, E525G, L855R, R929C, E1021K, and E1025G, on the PI3Kδ protein sequence. A non-functional endogenous *PIK3CD* allele can comprise at least one mutation not causing APDS1 but causing other disorders like refractory immune thrombocytopenia, inflammatory bowel disease and susceptibility to infection. The mutations in the *PIK3CD* locus associated with the other, non-APDS1, disorders include the mutations Q170Vfs*41*, V552Sfs*26, Q721*, R821H, N853Gfs*20 on the PI3Kδ protein sequence. In contrast, a "functional endogenous *PIK3CD* allele" encodes a functional PI3Kδ protein exhibiting the activity of the PI3Kδ protein of SEQ ID NO: 46. Generally, once integrated in the endogenous *PIK3CD* locus, said exogenous sequence allows the expression of the PI3Kδ protein of SEQ ID NO: 46.

The engineered HSC or T-cell may be a primary cell. Primary cells are generally used in cell therapy as they are deemed more functional and less tumorigenic. In general, primary cells can be obtained from the patient suffering from APDS1 through a variety of methods known in the art, as for instance by leukapheresis techniques as reviewed by Schwartz J. et al. [Guidelines on the use of therapeutic apheresis in clinical practice-evidence-based approach from the Writing Committee of the American Society for Apheresis: the sixth special issue (2013) J. Clin. Apher. 28(3): 145-284]. For example, primary HSCs can be taken from bone marrow, and more particularly from the pelvis, at the iliac crest, using a needle or syringe. Alternatively, HSCs may be harvested from the circulating peripheral blood, while blood donors are injected with a HSC mobilizing agent, such as chemokine (C-X-C motif) receptor 4 (CXCR4) antagonists, such as AMD3100 (also known as Plerixafor and MOZOBIL (Genzyme, Boston, Mass.)), granulocyte-colony stimulating factor (G-CSF), and chemokine (C-X-C motif) ligand 2 (CXCL2, also referred to as GROβ), which induces cells to leave the bone marrow and circulate in the blood vessels. HSCs may also be harvested from cord blood.

According to some embodiments, the engineered HSC is a mammalian HSC, preferably is a human HSC.

According to some embodiments, the engineered HSC is at least CD34+.

According to some embodiments, the engineered HSC is at least CD34+, CD90+, and CD133+.

According to some embodiments, the engineered HSC is at least CD34+, CD38-, CD45RA-, CD90+, and CD133+.

According to some embodiments, the engineered HSC is at least CD34+, CD38-, and CD45RA-.

According to some embodiments, the engineered HSC is at least CD34+, CD38-, CD45RA-, CD90-, and CD133-.

According to some embodiments, the engineered T-cell is a mammalian T-cell, preferably is a human T-cell.

In order to improve the expression of the functional PI3Kδ polypeptide and to avoid homologous recombination with the endogenous PIK3CD coding sequence, said nucleic acid sequence encoding a PI3Kδ protein may be codon optimized. Thus, according to some embodiments, said nucleic acid sequence encoding a PI3Kδ protein is a codon optimized nucleic acid sequence.

With "codon optimized" it is meant that the polynucleotide sequence has been adapted for expression in the cells of a given vertebrate, such as a human or other mammal, by replacing at least one, or more than one, or a significant number, of codons with one or more codons that are more frequently used in the genes of that vertebrate. Accordingly, the nucleic acid sequence encoding a PI3Kδ protein can be tailored for optimal gene expression in a given organism based on codon optimization. Codon optimization can be done based on established codon usage tables, for example, at the "Codon Usage Database" available at http://www.kazusa.or.jp/codon/ (hosted by Kazusa DNA Research Institute, Japan), or other kind of computer algorithms. A non-limiting example is the OptimumGene PSO algorithm from GenScript^{®} which takes into consideration a variety of critical factors involved in different stages of protein expression, such as codon adaptability, mRNA structure, and various cis-elements in transcription and translation. By utilizing codon usage tables or other kind of computer algorithms, one of ordinary skill in the art can apply the frequencies to any given polypeptide sequence, and produce a nucleic acid fragment of a codon-optimized coding region which encodes the polypeptide, but which uses codons optimal for a given organism.

Preferably, codon optimization also includes the removal of any cryptic splicing site as well as any microRNA target site and/or a sequence which would generate a mRNA secondary structure impeding translation. Thus, according to some embodiments, said codon optimized nucleic acid sequence has been further optimized to remove at least one cryptic splicing site and/or to remove at least one microRNA target site and/or a sequence which would generate a mRNA secondary structure impeding translation. Computer algorithms allowing the prediction of cryptic splicing sites are well known to the skilled person, and include, for example, the splice prediction tool available at http://wangcomputing.com/assp/. Similarly, one of ordinary skill in the art can use established computer algorithms, such as the miRNA target prediction tool available at http://mirdb.org/, to identify miRNA target sites.

According to some embodiments, said codon optimized nucleic acid sequence of PIK3CD comprises the nucleic acid sequence consisting of the codon optimized nucleic acid sequences of exons 1 to 24 of *PIK3CD* or a portion thereof of at least 100 nucleotides, SEQ ID NO: 1 or a portion thereof of at least 100 nucleotides, SEQ ID NO: 2 or a portion thereof of at least 100 nucleotides, or a codon optimized sequence of Exon 24 of *PIK3CD* or a portion thereof of at least 100 nucleotides.

According to some embodiments, said codon optimized nucleic acid sequence of PIK3CD comprises the nucleic acid sequence of SEQ ID NO: 1.

Further, in order to facilitate the nuclear export, translation and stability of mRNA, the exogenous sequence comprises a polyA signal. Non-limiting examples of polyA signals include polyA signals from SV40, hGH (human Growth Hormone), bGH (bovine Growth Hormone), and rbGlob (rabbit beta-globin). According to some embodiments, said polyA signal comprises the nucleic acid sequence of SEQ ID NO: 16 or nucleic acid sequence of SEQ ID NO: 17.

Further, in order to facilitate purification, detection and/or enrichment of the engineered PIK3CD-edited cells disclosed herewith, said exogenous polynucleotide sequence can comprise a PIK3CD sequence as described herewith, as well as a sequence encoding a surface marker such as the Low-affinity Nerve-Growth-Factor Receptor (LNGFR) or its derivatives including C-terminal deleted LNGFR, the Epidermal Growth Factor Receptor (EGFR) or the truncated human Epidermal Growth Factor Receptor (EGFRt). For instance, the low affinity nerve growth factor (NGF) receptor (LNGFR) or a C-terminal-deleted LNGFR version (DLNGFR) is a useful cell surface marker for the detection and purification of primitive and committed hematopoietic progenitor cells as described in Fehse et al. (Hum Gene Ther. (1997) 8:1815-1824) and (McCowage et al. Exp Hematol. (1998) 6:288-298). A sequence encoding a LNGFR or its derivatives useful in the present invention comprises SEQ ID NO: 21. In the present application, the term "LNGFR" will be used to designate the full-length LNGFR and its derivatives including the C-terminal deleted LNGFR form.

Also provided is an engineered HSC or T-cell, as described herewith, comprising an exogenous polynucleotide sequence comprising the sequence of a functional *PIK3CD* gene or a portion thereof of at least 100 nucleotides, said exogenous polynucleotide sequence being integrated in the cell's genome in an endogenous *PIK3CD* allele comprising at least one mutation causing APDS1, and further comprising an exogenous polynucleotide sequence comprising the sequence of a functional *PIK3CD* gene or a portion thereof of at least 100 nucleotides, said exogenous polynucleotide sequence being integrated in the cell's genome in an endogenous *PIK3CD* allele comprising no APDS1-associated mutation.

When an engineered HSC or T-cell as described herewith comprises two exogenous sequences, the nucleic acid sequences of the functional *PIK3CD* gene or portion thereof comprised in the two exogenous sequences do not need to be identical. In preferred embodiments, the nucleic acid sequences of the PIK3CD portion of the two exogenous sequences differ so as to reduce homologous recombination between the two exogenous sequences still encode the same amino acid sequences.

In further particular embodiments of said engineered HSC or T-cell, each of the two exogenous polynucleotide sequences further comprises a sequence encoding a surface marker, wherein the surface markers are different.

In a particular aspect, the engineered PIK3CD-edited HSCs or T-cells disclosed herewith comprise:
(a) an exogenous polynucleotide sequence comprising the sequence of a functional *PIK3CD* gene (or a portion thereof of at least 100 nucleotides) and a sequence encoding a first surface marker, said sequences being integrated in the cell's genome in an endogenous *PIK3CD* allele comprising at least one APDS1-associated mutation; and
(b) an exogenous polynucleotide sequence comprising the sequence of a functional *PIK3CD* gene or a portion thereof of at least 100 nucleotides and a sequence encoding a second surface marker, said sequences being integrated in the cell's genome in an endogenous *PIK3CD* allele comprising no APDS1-associated mutation;
wherein said first and second surface markers are different.

In a still preferred aspect of the above, the sequence of a functional *PIK3CD* gene or a portion thereof of at least 100 nucleotides comprised in each of the two exogenous polynucleotide sequences are different to avoid homologous recombination between these sequences.

The present invention further provides a population of cells comprising engineered HSCs orT-cells, more specifically PIK3CD-edited cells, as described herewith.

Preferably, said population of cells comprises at least about 1%, such as at least 2%, at least 4%, at least 5%, at least 6%, at least 7%, at least 8%, at least 9%, at least 10%, at least 15%, at least 20%, at least 25%, at least 30%, at least 35%, or at least 40% of said engineered HSCs or T-cells.

According to some embodiments, at least 20%, such as at least 30% or at least 40%, of the total cells of the population of cells are said engineered HSCs or T-cells.

According to some embodiments, at least 50%, such as at least 60% or at least 70%, of the total cells of the population of cells are engineered *PIK3CD* -edited HSCs or T-cells.

According to some embodiments, at least 80%, such as at least 90% or at least 95%, of the total cells of the population of cells are said engineered PIK3CD-edited HSCs or T-cells.

The population of cells according to the present invention may comprise long-term repopulating HSCs (LT-HSCs). According to some embodiments, at least 5%, at least 10%, at least 15%, at least 20%, at least 25% or at least 30%, of said LT-HSCs are engineered PIK3CD-edited HSCs.

The present invention further provides engineered PIK3CD -edited HSCs or T-cells as well as populations of engineered engineered PIK3CD-edited HSCs orT-cells obtainable by any of the production methods disclosed herein.

### Means and method for gene editing according to the invention

The present invention further provides means and methods for genetically modifying HSCs or T-cells involving gene editing reagents that specifically target an endogenous *PIK3CD* locus, in particular at least a *PIK3CD* allele comprising at least one APDS1-associated mutation, thereby allowing the restoration of the normal cellular phenotype. Targeted (i.e. site-directed) integration to achieve gene repair is suitably done by using sequence-specific reagents inducing DNA cleavage, such as a rare-cutting endonuclease or nickase, and exogenous polynucleotide donor templates bearing homology to the target site and comprising the corrective sequence. Targeted integration can also been achieved using sequence-specific reagents inducing transposition such as described by Owens et al. (Nucleic Acids Res. 2013, 41(19): 9197-207), Voigt et al. (Molecular Therapy 2012, 20(10): 1852-1862) or Bhatt et al. (Nucleic Acids Res. 2019, 47(15): 8126-8135).

The present invention thus provides sequence specific reagents inducing DNA cleavage that are capable of targeting and cleaving a sequence at any position within the endogenous *PIK3CD* locus.

Non-limiting examples of a "sequence-specific reagent inducing DNA cleavage" according to the invention include reagents that have nickase or endonuclease activity. The sequence-specific reagent can be a chimeric polypeptide comprising a DNA binding domain and another domain displaying catalytic activity. Such catalytic activity can be for instance a nuclease to perform gene inactivation, or nickase or double nickase to preferentially perform gene insertion by creating cohesive ends to facilitate gene integration by homologous recombination, or to perform base editing as described in Komor et al. (Nature 2016, 533(7603), 420-424).

In general, the sequence specific reagents of the present invention have the ability to recognize and bind a "target sequence" within an endogenous *PIK3CD* locus in an HSC or T-cell. The "target sequence" which is recognized and bound by the sequence specific reagents is usually selected to be rare or unique in the *PIK3CD* locus, as can be determined using software and data available from human genome databases, such as http://www.ensembl.org/index.html. Such "target sequence" is preferably one set forth in polynucleotide sequence of SEQ ID NO: 3 within Exon 8 of *PIK3CD,* SEQ ID NO: 6 within Exon 17 of *PIK3CD,* or SEQ ID NO: 9 within Exon 24 of *PIK3CD* gene.

Thus, according to the present invention, sequence-specific reagents inducing DNA cleavage that are capable of targeting and cleaving a sequence as set forth in SEQ ID NO: 3 within Exon 8 of *PIK3CD,* SEQ ID NO: 6 within Exon 17 of *PIK3CD,* or SEQ ID NO: 9 within Exon 24 of *PIK3CD* gene, are provided.

According to some embodiments, the sequence-specific reagent inducing DNA cleavage is a rare-cutting endonuclease. "Rare-cutting endonucleases" are sequence-specific endonuclease reagents of choice, insofar as their recognition sequences generally range from 10 to 50 successive base pairs, preferably from 12 to 30 bp, and more preferably from 14 to 20 bp.

According to some embodiments, said rare-cutting endonuclease is an "engineered" or "programmable" rare-cutting endonuclease, such as a homing endonuclease as described for instance by Arnould et al. (WO2004067736), a zing finger nuclease (ZFN) as described, for instance, by Urnov et al. (Nature 2005, 435, 646-651), a TALE-nuclease as described, for instance, by Mussolino et al. (Nucl. Acids Res. 2011, 39(21), 9283-9293), or a MegaTAL nuclease as described, for instance by Boissel et al. (Nucleic Acids Research 2013, 42 (4), 2591-2601).

Due to their higher specificity, TALE-nucleases ("TALEN") have proven to be particularly appropriate sequence specific nuclease reagents for therapeutic applications, especially under heterodimeric forms, i.e. working by pairs with a "right" monomer (also referred to as "5‴ or "forward") and 'left" monomer (also referred to as "3‴ or "reverse") as reported for instance by Mussolino et al. (Nucl. Acids Res. 2014, 42(10), 6762-6773). Thus, according to some preferred embodiments, said rare-cutting endonuclease is a TALE-nuclease.

Thus, is provided herewith a TALE-Nuclease heterodimer targeting an endogenous *PIK3CD* locus in an HSC or T-cell.

Particularly, it is provided herewith a TALE-Nuclease heterodimer targeting the polynucleotide sequence of SEQ ID NO: 3 within Exon 8 of *PIK3CD.*

According to some embodiments, said TALE-Nuclease heterodimer comprises a first monomer comprising the amino acid sequence of SEQ ID NO: 40 or a variant thereof comprising an amino acid sequence having at least 80% sequence identity with SEQ ID NO: 40, and a second monomer comprising the amino acid sequence of SEQ ID NO: 41 or a variant thereof comprising an amino acid sequence having at least 80% sequence identity with SEQ ID NO: 41, wherein the heterodimer targets the sequence of SEQ ID NO: 3. In particular, said first monomer targets the sequence of SEQ ID NO: 4 and said second monomer targets the sequence of SEQ ID NO: 5.

According to some embodiments, said TALE-Nuclease heterodimer comprises a first monomer comprising the amino acid sequence of SEQ ID NO: 40 and a second monomer comprising the amino acid sequence SEQ ID NO: 41.

These monomers and variants thereof are designed to form a heterodimer binding to the target polynucleotide sequence SEQ ID NO: 3 and comprise the RVD sequence NN-NI-NI-HD-NN-HD-HD-NN-NI-HD-NN-NI-NN-HD-NN-NG# and RVD sequence HD-HD-HD-NI-HD-HD-NG-NI-NG-HD-HD-HD-NI-NN-NN-NG#, respectively. The cleavage occurs within the target sequence in the endogenous *PIK3CD* locus.

Also provided herewith is a TALE-Nuclease heterodimer targeting the polynucleotide sequence of SEQ ID NO: 6 within Exon 17 of *PIK3CD.*

According to some embodiments, said TALE-Nuclease heterodimer comprises a first monomer comprising the amino acid sequence of SEQ ID NO: 42 or a variant thereof comprising an amino acid sequence having at least 80% sequence identity with SEQ ID NO: 42, and a second monomer comprising the amino acid sequence of SEQ ID NO: 43 or a variant thereof comprising an amino acid sequence having at least 80% sequence identity with SEQ ID NO: 43, wherein the heterodimer targets the sequence of SEQ ID NO: 6. In particular, said first monomer targets the sequence of SEQ ID NO: 7 and said second monomer targets the sequence of SEQ ID NO: 8.

According to some embodiments, said TALE-Nuclease heterodimer comprises a first monomer comprising the amino acid sequence of SEQ ID NO: 42 and a second monomer comprising the amino acid sequence SEQ ID NO: 43.

These monomers and variants thereof are designed to form a heterodimer binding to the target polynucleotide sequence SEQ ID NO: 6 and comprise the RVD sequence NN-NI-NG-NN-HD-NI-HD-NG-NG-NN-NG-NN-HD-NI-NG-NG# and RVD sequence NN-NN-NN-NI-NN-NI-NN-NN-NN-HD-HD-NG-HD-NG-NI-NG#, respectively. The cleavage occurs within the target sequence in the endogenous *PIK3CD* locus.

Also provided herewith is a TALE-Nuclease heterodimer targeting the polynucleotide sequence of SEQ ID NO: 9 within Exon 24 of *PIK3CD.*

According to some embodiments, said TALE-Nuclease heterodimer comprises a first monomer comprising the amino acid sequence of SEQ ID NO: 44 or a variant thereof comprising an amino acid sequence having at least 80% sequence identity with SEQ ID NO: 44, and a second monomer comprising the amino acid sequence of SEQ ID NO: 45 or a variant thereof comprising an amino acid sequence having at least 80% sequence identity with SEQ ID NO: 45, wherein the heterodimer targets the sequence of SEQ ID NO: 9. In particular, said first monomer targets the sequence of SEQ ID NO: 10 and said second monomer targets the sequence of SEQ ID NO: 11.

According to some embodiments, said TALE-Nuclease heterodimer comprises a first monomer comprising the amino acid sequence of SEQ ID NO: 44 and a second monomer comprising the amino acid sequence SEQ ID NO: 45.

These monomers and variants thereof are designed to form a heterodimer binding to the target polynucleotide sequence SEQ ID NO: 9 and comprise the RVD sequence NN-NN-HD-NI-HD-NG-NN-NN-NN-NN-NI-NI-NI-NI-HD-NG# and RVD sequence NN-NG-NG-NI-NI-NI-HD-NG-NG-HD-NI-HD-NG-HD-NN-NG#, respectively. The cleavage occurs within the target sequence, at the positions encoding the E1021K mutation, in the endogenous *PIK3CD* locus.

In one embodiment, the nuclease specific reagent is a Transcription Activator-Like Effector derived protein that allows to efficiently target and/or process chemically modified nucleic acids. More particularly, the nuclease specific reagent is a TALE derived protein comprising Repeat Variable-Diresidue (RVD) that allow to efficiently detect, target and/or process nucleic acids with chemical modifications such as alkylation, in particular cytosine methylation as a non-limiting example, as described in WO2013/140250. In a particular embodiment, the nuclease specific reagent is a TALE derived protein comprising RVDs X* as an entity capable of efficient binding of chemically modified base, wherein X represents one amino acid residue selected from the group of A, G, V, L, I, M, S, T, C, P, D, E, F, Y, W, Q, N, H, R and K and * represents a gap in position 13 of the RVD.

Repeat Variable Diresidue (RVD) is included in one repeat module or sequence responsible for the binding of a nucleic acid base in a nucleic acid target sequence at the level of variable amino acids located at positions 12 and 13 (i.e. Repeat Variable Di residues, RVD).

In a particular embodiment, said RVD region responsible for the binding of a nucleic acid base comprises any known amino acid residues in positions 12 and 13. In a preferred embodiment, RVDs comprise one amino acid residue from the group consisting of A, G, V, L, I, M, S, T, C, P, D, E, F, Y, W, Q, N, H, R and K in position 12 according to amino acid one-letter code. In a preferred embodiment, RVDs responsible for the binding of a modified nucleic acid base comprise a gap in position 13, more particularly RVDs are X*, preferably N*, Q*, T* or H* and are capable of efficient binding of chemically modified base, wherein X represents one amino acid residue selected from the group of A, G, V, L, I, M, S, T, C, P, D, E, F, Y, W, Q, N, H, R and K and * represents a gap in position 13 of the RVD. In a still other embodiment, RVDs at position 12 and position 13 are NA, HA, or RG.

Said RVD of the TALE derived protein as described above is capable of binding a modified nucleotide comprising a base different from the classical purine and pyrimidine bases, i.e respectively Adenine, Guanine and Cytosine, Uracil and Thymine. In another aspect, said chemically modified nucleic acid base recognized by the RVD of the TALE derived protein as described above is a nucleotide comprising one or several additional chemical groups such as alkyl or hydroxyl as non-limiting example. Said additional group may be a methyl group which refers to the transfer of one carbon group on a nucleotide. Alkylation refers to the transfer of a long chain carbon group. In another embodiment, said chemically modified nucleotide comprises a 5-methyl cytosine base. In another embodiment, said modified nucleic acid base comprises a base selected from the group consisting of 5-hydroxymethylcytosine, 5-formylcytosine and 5-carboxylcytosine. In another embodiment, said RVD of the TALE derived protein as described above is capable of binding DNA sequences comprising molecular lesions such as a non-limiting example pyrimidine dimers formed from cytosine or thymine bases via photochemical reactions.

The repeat sequence or repeat module of TALE derived protein as described herewith can comprise variable residues in positions 12 and 13 as mentioned above, as well as one or several additional mutations in one or several of the 30 to 42 amino acids constituting said RVD, more preferably 33 to 35 amino acids, again more preferably 33 or 34 amino acids. By mutations are encompassed substitutions toward any natural amino acids from the group consisting of A, G, V, L, I, M, S, T, C, P, D, E, F, Y, W, Q, N, H, R and K according to amino acid one-letter code, but also insertions and deletions of one or several amino acid residues.

In other words, a nuclease specific reagent as described herewith include a TALE derived protein comprising one repeat module or sequence responsible for the binding of a modified nucleic acid base in a nucleic acid target sequence at the level of variable amino acids located at positions 12 and 13 (i.e. Repeat Variable Di residues, RVD). In particular, the repeat sequence or module of a TALE comprises a RVD comprising X*, preferably N*, Q*, T* or H* for binding chemical modified base nucleic acid wherein X represents one amino acid residue selected from the group of A,G, V, L, I, M, S, T, C, P, D, E, F, Y, W, Q, N, H, R and K and * represents a gap in position 13 of the RVD.

In another embodiment, a nuclease specific reagent as described herewith include a TALE derived protein comprising a TALE binding domain specific for a nucleic acid target sequence comprising a plurality of TALE repeat sequences comprising each one a Repeat Variable Diresidue region (RVD) which is responsible for the binding of one specific nucleic acid base in said nucleic acid target sequence and wherein said TALE DNA binding domain comprises one or more RVD comprising X*, preferably N*, Q*, T* or H* for binding chemically modified nucleic acid base, wherein X represents one amino acid residue selected from the group of A,G, V, L, I, M, S, T, C, P, D, E, F, Y, W, Q, N, H, R and K and * represents a gap in position 13 of the RVD. In a preferred embodiment, said repeat domain comprises between 8 and 30 repeat sequences derived from a TALE, more preferably between 8 and 20, again more preferably 5, 6, 7, 8, 9, 10, 11, 12, 13, 14, 15, 16, 17, 18, 19, 20 repeat sequences.

Thus, also provided is a TALE-Nuclease heterodimer as described herewith comprising repeat sequences comprising RVDs, in particular at position 12 and/or position 13, which are X*, wherein X represents one amino acid residue selected from the group consisting of A, G, V, L, I, M, S, T, C, P, D, E, F, Y, W, Q, N, H, R and K, preferably N, Q, T or H, and * represents a deletion in position 13 of the RVD; or comprising repeat sequences comprising RVDs, in particular at position 12 and/or position 13, which are NA, HA, or RG.

In particular, is provided a TALE-Nuclease heterodimer as described herewith, wherein said TALE-Nuclease heterodimer targets the polynucleotide sequence of SEQ ID NO: 9 within Exon 24 of *PIK3CD* gene, and comprises a pair of monomers comprising repeat sequences comprising RVDs, in particular at position 12 and/or position 13, which are N*or H*, wherein * represents a deletion in position 13 of the RVD.

According to some embodiments, the rare-cutting endonuclease is an RNA guided endonuclease, such as Cas9 or Cpf1, to be used in conjunction with a RNA-guide as per, inter alia, the teaching by Doudna et al. (Science 2014, 346 (6213): 1077) and Zetsche et al. (Cell 2015, 163(3): 759-771). The RNA-guide is designed to hybridize a target sequence.

According to some embodiments, the sequence-specific reagent inducing DNA cleavage is a nickase.

The present invention further provides an isolated nucleic acid encoding the sequence-specific reagent inducing DNA cleavage of the present invention.

According to some embodiments, said isolated nucleic acid is a mRNA.

The present invention further provides a vector, such as a viral vector, and more specifically a non-integrative viral vector such as an AAV or IDLV vector, comprising a polynucleotide sequence encoding the sequence-specific reagent inducing DNA cleavage of the present invention.

The present invention also provides the *ex vivo* use of the sequence specific reagent inducing DNA cleavage as described herewith, the isolated nucleic acid of the invention encoding same or the vector encoding same in gene editing HSCs or T-cells, notably HSCs or T-cells of a patient suffering from APDS1.

The present invention further provides an exogenous sequence as defined above in the form of an isolated nucleic acid or vector comprising same. The isolated nucleic acid or vector will function as a polynucleotide donor template.

In order to facilitate targeted (i.e. site-directed) integration of the exogenous sequence via homologous recombination, said exogenous sequence is placed between a left and/or a right homology sequence having at least 80% sequence identity with a part of the endogenous *PIK3CD* locus. Specifically, the exogenous sequence is placed between a left homologous region at its 5' end and a right homologous region at its 3' end, homologous regions being relative to parts of *PIK3CD* locus.

In order to facilitate targeted (i.e. site-directed) integration of the exogenous sequence via NHEJ repair mechanism at the cleaved locus, said exogenous sequence may comprise microhomologies, i.e. short homologous DNA sequences.

The present invention further provides an isolated nucleic acid comprising said exogenous sequence.

The present invention further provides a vector comprising said exogenous sequence, such as a viral vector, and more specifically a non-integrative viral vector such as an AAV or IDLV vector, comprising said exogenous sequence. Thus, according to some embodiments, the vector is an AAV vector, preferably an AAV6 vector. AAV vectors, and especially AAV6, are particularly suited for transduction of polynucleotide donor templates into cells and to perform integration by homologous recombination directed by rare-cutting endonucleases as described for instance by Sather et al. (Science translational medicine, 2015, 7(307): 307ra156).

The present invention also provides the *ex vivo* use of an isolated nucleic acid or vector of the invention, comprising the exogenous sequence as defined herein, in gene editing HSCs or T-cells, notably HSCs or T-cells of a patient suffering from APDS1.

The present invention also provides the *ex vivo* use of the sequence specific reagent inducing DNA cleavage of the invention, the polynucleotide of the invention encoding same or the vector encoding same in combination with an isolated nucleic acid or vector of the invention, comprising the exogenous sequence as defined herein, for use in gene editing HSCs or T-cells, notably HSCs or T-cells of a patient suffering from APDS1.

Provided herewith is a kit comprising:
(i) at least one isolated nucleic acid or vector comprising an exogenous sequence as defined herein and, optionally, a nucleic acid sequence encoding a surface marker, and
(ii) a sequence-specific reagent inducing DNA cleavage as described herewith.

According to some embodiments, said kit comprises an isolated nucleic acid or vector comprising the nucleic acid sequence SEQ ID NO: 1 and, optionally, a nucleic acid sequence encoding a surface marker; and at least one isolated nucleic acid or vector comprising a nucleic acid sequence encoding the amino acid sequences of SEQ ID NO: 40 and/or SEQ ID NO: 41.

According to some embodiments, said kit comprises an isolated nucleic acid or vector comprising the nucleic acid sequence SEQ ID NO: 2 and, optionally, a nucleic acid sequence encoding a surface marker; and at least one isolated nucleic acid or vector comprising a nucleic acid sequence encoding the amino acid sequences of SEQ ID NO: 42 and/or SEQ ID NO: 43.

According to some embodiments, said kit comprises an isolated nucleic acid or vector comprising a codon optimized sequence of Exon 24 of *PIK3CD* and, optionally, a nucleic acid sequence encoding a surface marker; and at least one isolated nucleic acid or vector comprising a nucleic acid sequence encoding the amino acid sequences of SEQ ID NO: 44 and/or SEQ ID NO: 45.

The present invention also provides the *ex vivo* use of the kits of the invention, in gene editing HSCs or T-cells, notably HSCs or T-cells of a patient suffering from APDS1.

The present invention further provides a method of preparing engineered PIK3CD-edited haematopoietic stem cells or T-cells comprising introducing into the cells to be engineered which have a non-functional endogenous *PIK3CD* allele comprising at least one APDS1-associated mutation, said method comprising:
(i) at least one sequence specific reagent inducing DNA cleavage that is capable of specifically targeting and cleaving a sequence within an endogenous *PIK3CD* locus, in at least a non-functional *PIK3CD* endogenous allele, in said cells;
(ii) at least one isolated nucleic acid or vector comprising an exogenous sequence as defined herewith, wherein said exogenous sequence is placed between a left homologous region at its 5' end and a right homologous region at its 3' end, homologous regions being relative to the targeted portion of the *PIK3CD* gene;
whereby engineered PIK3CD-edited HSCs or T-cells are obtained, which comprise a disrupted non-functional *PIK3CD* allele and comprise a nucleic acid sequence encoding a functional PI3Kδ protein.

The method has been particularly designed to obtain engineered PIK3CD-edited HSCs or T-cells for the treatment of a patient suffering from APDS1 by gene therapy, more particularly by integrating a corrective coding sequence of PIK3CD, or a portion thereof of at least 100 nucleotides, at a non-functional endogenous *PIK3CD* locus using the exogenous sequence and the sequence-specific reagent inducing DNA cleavage described herein.

The method is preferably practiced *ex vivo* to obtain stably engineered PIK3CD-edited HSCs or T-cells. The resulting engineered HSCs or T-cells can be then engrafted into a patient in need thereof for a long term *in-vivo* production of engineered cells that will comprise said exogenous sequence as detailed herein.

According to some embodiments, said sequence specific reagent of (i) comprises a nucleic acid sequence encoding the amino acid sequences of SEQ ID NO: 40 and SEQ ID NO: 41, and said nucleic acid or vector of (ii) comprises an exogenous sequence comprising the nucleic acid sequence SEQ ID NO: 1 and optionally a nucleic acid sequence encoding a surface marker.

According to some embodiments, said sequence specific reagent of (i) comprises a nucleic acid sequence encoding the amino acid sequences of SEQ ID NO: 42 and SEQ ID NO: 43, and said nucleic acid or vector of (ii) comprises an exogenous sequence comprising the nucleic acid sequence SEQ ID NO: 2 and optionally a nucleic acid sequence encoding a surface marker.

According to some embodiments, said sequence specific reagent of (i) comprises a nucleic acid sequence encoding the amino acid sequences of SEQ ID NO: 44 and SEQ ID NO: 45, and said nucleic acid or vector of (ii) comprises an exogenous sequence comprising a codon optimized sequence of Exon 24 of *PIK3CD* and optionally a nucleic acid sequence encoding a surface marker.

According to some embodiments, the sequence-specific reagent inducing DNA cleavage, such as a rare-cutting endonuclease, is transiently expressed or delivered in the cells, meaning that said reagent is not supposed to integrate into the genome or persist over a long period of time, such as would be the case of RNA, more particularly mRNA, proteins or complexes mixing proteins and nucleic acids (eg: Ribonucleoproteins). Preferably, the sequence-specific reagent inducing DNA cleavage, such as a rare-cutting endonuclease, is introduced into the cell in the form of a nucleic acid molecule, such as a DNA or RNA molecule, preferably mRNA molecule, encoding said sequence-specific reagent, and will be expressed by the transfected cell. A sequence-specific reagent inducing DNA cleavage, such as a rare-cutting endonuclease, under mRNA form is preferably synthetized with a cap to enhance its stability according to techniques well known in the art, as described, for instance, by Kore et al. (J Am Chem Soc. (2009) 131(18): 6364-5).

According to some embodiments, said sequence-specific reagent inducing DNA cleavage is introduced into said HSCs or T-cells via a vector.

According to some embodiments, said sequence-specific reagent inducing DNA is introduced into said HSCs or T-cells as mRNA by electroporation.

According to some embodiments, said sequence-specific reagent inducing DNA cleavage is introduced into said HSCs or T-cells via nanoparticles, preferably nanoparticles which are coated with ligands, such as antibodies, having a specific affinity towards a HSC or T-cell surface protein, such as CD105 (Uniprot #P17813). Preferred nanoparticles are biodegradable polymeric nanoparticles in which the sequence specific reagent under polynucleotide form is complexed with a polymer of polybeta amino ester and coated with polyglutamic acid (PGA).

According to some embodiments, methods of non-viral delivery of the exogenous sequence and/or the sequence-specific reagent inducing DNA cleavage can be used such as electroporation, lipofection, microinjection, biolistics, virosomes, liposomes, immunoliposomes, polycation or lipid:nucleic acid conjugates, naked DNA, naked RNA, capped RNA, artificial virions, and agent-enhanced uptake of DNA. Sonoporation using, e.g., the Sonitron 2000 system (Rich-Mar) can also be used for delivery.

According to some embodiments, electroporation steps can be used to transfect cells. In general, electroporation steps that are used to transfect cells are typically performed in closed chambers comprising parallel plate electrodes producing a pulse electric field between said parallel plate electrodes greater than 100 volts/cm and less than 5,000 volts/cm, substantially uniform throughout the treatment volume such as described in WO/2004/083379, especially from page 23, line 25 to page 29, line 11. One such electroporation chamber preferably has a geometric factor (cm-1) defined by the quotient of the electrode gap squared (cm2) divided by the chamber volume (cm3), wherein the geometric factor is less than or equal to 0.1 cm-1, wherein the suspension of the cells and the sequence-specific reagent is in a medium which is adjusted such that the medium has conductivity in a range spanning 0.01 to 1.0 milliSiemens. In general, the suspension of cells undergoes one or more pulsed electric fields. With the method, the treatment volume of the suspension is scalable, and the time of treatment of the cells in the chamber is substantially uniform.

The method may further involve the use of one or more polypeptides that enhances homologous recombination and/or viability of HSCs, such as a dominant negative p53 fragment, such as GSE56, and/or a p53-binding protein 1 inhibitor or a dominant-negative form of tumour protein p53-binding protein.

According to some embodiments, said HSCs or T-cells are isolated from a tissue sample from a human donor, such as a human patient suffering from a disorder related to *PIK3CD,* including APDS1 and other non-APDS1 disorders such as refractory immune thrombocytopenia, inflammatory bowel disease and susceptibility to infection.

For example, said human patient suffering from APDS1 can have at least one PIK3CD mutation selected from the group consisting of E81K, G124D, N334K, R405C, C416R, Y524N, Y524S, Y524D, E525A, E525K, E525G, L855R, R929C, E1021K, and E1025G, on the PI3K protein sequence.

For example, said human patient suffering from non-APDS1 disorders related to *PIK3CD* can have at least one PIK3CD mutation selected from the group consisting of Q170Vfs*41*, V552Sfs*26, Q721*, R821H, N853Gfs*20, on the PI3K protein sequence (where * indicates premature stop and fs indicates frameshift).

According to some embodiments, said tissue sample is a peripheral blood sample.

According to some embodiments, said tissue sample is obtained from a human donor who has been administered with at least one HSC mobilizing agent, such as G-CSF and/or plerixafor, prior to obtaining the tissue sample.

The present invention further provides a population of cells comprising HSCs or T-cells comprising a sequence specific reagent inducing DNA cleavage of the present invention and an exogenous sequence as defined herein.

According to some embodiments, said population comprises HSCs or T-cells comprising a TALE-Nuclease heterodimer as described herewith and an exogenous sequence as defined herein.

### Activation and gene editing of HSCs

For mobilised peripheral blood (MPB) leukapheresis, CD34+ cells are generally processed and enriched using immunomagnetic beads such as CliniMACS. Purified CD34+ cells are seeded on culture bags at 1 × 10⁶ cells/ml in serum-free medium in the presence of cell culture grade Stem Cell Factor (SCF), preferably 300 ng/ml, preferably with FMS-like tyrosine kinase 3 ligand (FLT3L) 300 ng/ml, and Thrombopoietin (TPO), preferably around 100 ng/ml and further interleukine IL-3, preferably more than 60 ng/ml (all from CellGenix) during between preferably 12 and 24 hours before being transferred to an electroporation buffer comprising mRNA encoding the sequence specific reagent. Upon electroporation, the cells are preferably cryopreserved.

### Activation and expansion of T-cells

Whether prior to or after genetic modification, the T-cells as described herewith can be activated or expanded, even if they can activate or proliferate independently of antigen binding mechanisms. T-cells, in particular, can be activated and expanded using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005. T-cells can be expanded *in vitro* or *in vivo.* T-cells are generally expanded by contact with an agent that stimulates a CD3 TCR complex and a co-stimulatory molecule on the surface of the T-cells to create an activation signal for the T-cell. For example, chemicals such as calcium ionophore A23187, phorbol 12-myristate 13-acetate (PMA), or mitogenic lectins like phytohemagglutinin (PHA) can be used to create an activation signal for the T-cell.

As non-limiting examples, T-cell populations may be stimulated *in vitro* such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T-cells, a ligand that binds the accessory molecule is used. For example, a population of T-cells can be contacted with an anti-CD3 antibody and an anti-CD28 antibody, under conditions appropriate for stimulating proliferation of the T-cells. Conditions appropriate for T-cell culture include an appropriate media (e.g., Minimal Essential Media or RPMI Media 1640 or, X-vivo 5, (Lonza)) that may contain factors necessary for proliferation and viability, including serum (e.g., fetal bovine or human serum), interleukin-2 (IL-2), insulin, IFN-g , 1L-4, 1L-7, GM-CSF, -10, -2, 1L-15, TGFp, and TNF- or any other additives for the growth of cells known to the skilled artisan. Other additives for the growth of cells include, but are not limited to, surfactant, plasmanate, and reducing agents such as N-acetyl-cysteine and 2-mercaptoethanoi. Media can include RPMI 1640, A1M-V, DMEM, MEM, a-MEM, F-12, X-Vivo 1, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T-cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37°C) and atmosphere (e.g., air plus 5% CO₂). T-cells that have been exposed to varied stimulation times may exhibit different characteristics.

In another particular embodiment, said cells can be expanded by co-culturing with tissue or cells. Said cells can also be expanded in vivo, for example in the patient's blood after administrating said cell into the patient.

### Purification of the PIK3CD-edited cells

In some embodiments, the engineered PIK3CD-edited HSCs or T-cells described herewith have integrated in their genome at least one exogenous polynucleotide sequence comprising a PIK3CD sequence as described herewith and a nucleic sequence encoding a surface marker (e.g.; LNGFR including its derivatives like C-terminal deleted LNGFR, EGFR, EGFRt).

The presence of said surface marker at the surface of the PIK3CD-edited cells facilitates their purification, detection, tracking and/or enrichment.

Thus, is provided herewith a method of purifying engineered PIK3CD-edited HSCs or T-cells originating from a patient suffering from APDS1, which have integrated in their genome at least one exogenous polynucleotide sequence comprising a PIK3CD sequence and a nucleic acid sequence encoding a surface marker, said method comprising binding the engineered PIK3CD-edited HSCs or T-cells with a binding agent specific for said surface marker, wherein said binding agent is fixed to a support, and eluting the PIK3CD-edited HSCs or T-cells from the support.

Also provided herewith is, thus, a method of purifying engineered PIK3CD-edited HSCs or T-cells originating from a patient suffering from APDS1, comprising:
(i) providing a population of cells comprising the engineered HSCs or T-cells which have integrated in their genome at least one exogenous polynucleotide sequence comprising a PIK3CD sequence and a nucleic acid sequence encoding a surface marker as described herewith;
(ii) binding said cells with a binding agent specific for said surface marker, wherein said binding agent is fixed to a support; and
(iii) eluting the engineered HSCs or T-cells from the support;
wherein the eluted product is enriched in PIK3CD-edited HSCs or T-cells.

In some advantageous embodiments, the engineered PIK3CD-edited cells provided herewith comprise (i) an exogenous polynucleotide sequence comprising the sequence of a functional *PIK3CD* gene, or a portion thereof of at least 100 nucleotides, and a nucleic acid sequence encoding a first surface marker, wherein said exogenous polynucleotide is integrated in the cell's genome in an endogenous *PIK3CD* allele comprising at least one APDS1-associated mutation; and (ii) an exogenous polynucleotide sequence comprising the sequence of a functional *PIK3CD* gene, or a portion thereof of at least 100 nucleotides, and a nucleic acid sequence encoding a second surface marker, wherein said exogenous polynucleotide sequence is integrated in the cell's genome in an endogenous *PIK3CD* allele comprising no APDS1-associated mutation; wherein the first and second surface markers of (i) and (ii) are different.

Thus, is also provided herewith a method of purifying engineered PIK3CD-edited HSCs or T-cells originating from a patient suffering from APDS1, comprising:
(i) providing a population of cells comprising the engineered HSCs or T-cells expressing a first and a second surface markers as described above;
(ii) binding said engineered HSCs or T-cells with a binding agent specific for a first surface marker and with a binding agent specific for a second surface marker, wherein said binding agents are fixed to a support and wherein said first and second surface markers are different;
(iii) eluting the engineered HSCs or T-cells from the support;
wherein the eluted product is enriched in PIK3CD-edited HSCs or T-cells comprising (a) an exogenous polynucleotide sequence comprising the sequence of a functional *PIK3CD* gene (or a portion thereof of at least 100 nucleotides) and a sequence encoding a first surface marker, said sequences being integrated in the cell's genome in an endogenous *PIK3CD* allele comprising at least one APDS1-associated mutation; and (b) an exogenous polynucleotide sequence comprising the sequence of a functional *PIK3CD* gene or a portion thereof of at least 100 nucleotides and a sequence encoding a second surface marker, said sequences being integrated in the cell's genome in an endogenous *PIK3CD* allele comprising no APDS1-associated mutation.

Purification methods based on sorting cells according to their ability to bind to binding agents (e.g.; antibodies) specific for a marker expressed at their cell surface are well known to the skilled person and include, for instance, those based on Magnetic-Activated Cell Sorting (MACS) or Fluorescence Activated Cell Sorting (FACS), as described e.g. in Bacon et al (Acta Biomaterialia, (2020) 112: 29-51), Hu et al. (Front. Cell Dev. Biol. (2016) 4:116), Almeida et al Pathobiology (2014) 81:261-275).

### Therapeutic methods, compositions and uses of the invention

The present invention described above allows producing engineered HSCs or T-cells or populations comprising such engineered cells in which the initially defective endogenous *PIK3CD* gene sequence causing APDS1 has been replaced by a corrective PIK3CD sequence or portion thereof, thereby restoring the normal cellular phenotype by preventing expression of the non-fucntional hyper-activated PI3Kδ protein and enabling expression of a functional PI3Kδ protein. This makes the engineered HSCs or T-cells, respectively the population of HSCs or T-cells of the present invention particularly useful in the treatment of APDS1. These cells or population of cells may also be used in the manufacture of a medicament, such as a medicament for use in the treatment of APDS1.

The present invention thus provides the engineered HSCs or T-cells, respectively the population of HSCs orT-cells of the present invention for use in the treatment of APDS1.

The present invention thus provides the engineered HSCs, respectively the population of HSCs of the present invention for use in stem cell transplantation, such as bone marrow transplantation.

The present invention also provides a pharmaceutical composition comprising at least one engineered HSC or T-cell of the present invention, or a population of engineered hematopoietic stem cells (HSCs) or T-cells of the present invention, and a pharmaceutically acceptable excipient and/or carrier.

Suitable, such composition comprises the engineered HSC or T-cell, or the population of engineered hematopoietic stem cells (HSCs) or T-cells, in a therapeutically effective amount. An "effective amount" or "therapeutically effective amount" refers to that amount of a composition described herein which, when administered to a subject, is sufficient to provides a therapeutic or prophylactic benefit, i.e. aids in treating or preventing the disease. The amount of a composition that constitutes a "therapeutically effective amount" may vary depending on the cell preparations, the condition and its severity, the manner of administration, and the age of the subject to be treated, but can be determined routinely by one of ordinary skill in the art having regard to his own knowledge and to this disclosure.

The invention is thus more particularly drawn to a therapeutically effective population of engineered HSCs or T-cells, wherein at least 30%, preferably 50%, more preferably 80% of the cells in said population have been modified according to any one the methods described herein. Said therapeutically effective population of engineered HSCs or T-cells, as per the present invention, comprises cells with a corrected endogenous *PIK3CD* locus, allowing the expression of functional PI3Kδ protein and no non-functional hyper-activated PI3Kδ protein.

Suitable pharmaceutically acceptable excipients and carriers are well-known to the skilled person, and have been described in the literature, such as in Remington's Pharmaceutical Sciences, the Handbook of Pharmaceutical Additives or the Handbook of Pharmaceutical Excipients.

In some embodiments, the invention provides a cryopreserved pharmaceutical composition comprising: (a) a viable composition of engineered HSCs or T-cells (b) an amount of cryopreservative sufficient for the cryopreservation of the HSC or T-cells; and (c) a pharmaceutically acceptable carrier.

As used herein, "cryopreservation" refers to the preservation of cells by cooling to low sub-zero temperatures, such as (typically) 77 K or -196°C. (the boiling point of liquid nitrogen). At these low temperatures, any biological activity, including the biochemical reactions that would lead to cell death, is effectively stopped. Cryoprotective agents are often used at sub-zero temperatures to preserve the cells from damage due to freezing at low temperatures or warming to room temperature. The injurious effects associated with freezing can be circumvented by (a) use of a cryoprotective agent, (b) control of the freezing rate, and (c) storage at a temperature sufficiently low to minimize degradative reactions. Cryoprotective agents which can be used include but are not limited to dimethyl sulfoxide (DMSO), glycerol, polyvinylpyrrolidine, polyethylene glycol, albumin, dextran, sucrose, ethylene glycol, i-erythritol, D-Sorbitol, D-mannitol, D-sorbitol, i-inositol, D-lactose, choline chloride, amino acids, methanol, acetamide, glycerol monoacetate, and inorganic salts. In a preferred embodiment, DMSO is used, a liquid which is nontoxic to cells in low concentration. Being a small molecule, DMSO freely permeates the cell and protects intracellular organelles by combining with water to modify its freezability and prevent damage from ice formation. Addition of plasma (e.g., to a concentration of 20-25%) can augment the protective effect of DMSO. After the addition of DMSO, cells should be kept at 0-4°C. until freezing, since DMSO concentrations of about 1% are toxic at temperatures above 4°C.

The present invention provides the pharmaceutical composition of the present invention for use in the treatment of APDS1.

The present invention also provides the pharmaceutical composition of the present invention for use in in hematopoietic stem cell transplantation.

The present invention also provides an isolated nucleic acid or vector of the invention, comprising the exogenous sequence as defined herein, for use in the treatment of APDS1.

The present invention also provides the sequence specific reagent inducing DNA cleavage of the invention, the isolated nucleic acid of the invention encoding same or the vector encoding same for use in the treatment of APDS1.

The present invention also provides the sequence specific reagent inducing DNA cleavage of the invention, the isolated nucleic acid of the invention encoding same or the vector encoding same in combination with an isolated nucleic acid or vector of the invention, comprising the exogenous sequence as defined herein, for use in the treatment of APDS1.

The present invention also provides the kit of the invention for use in the treatment of APDS1.

The present invention also provides the sequence specific reagent inducing DNA cleavage of the invention, the polynucleotide of the invention encoding same or the vector encoding same in combination with an isolated nucleic acid or vector of the invention, comprising the exogenous sequence as defined herein, for use in gene editing *in vivo* in HSCs or T-cells, notably HSCs or T-cells of a patient suffering from APDS1.

The present invention further provides a method for treating APDS1 in a patient, such as a human patient, in need thereof, the method comprising administering an engineered PIK3CD-edited HSC or T-cell, a population of cells or a pharmaceutical composition, as described herewith, to said patient.

Also provided herewith is a method for haematopoietic stem cell transplantation in a patient, such as a human patient, in need thereof, the method comprising administering an engineered PIK3CD-edited HSC, in particular an autologous engineered PIK3CD-edited HSC, a population of cells or a pharmaceutical composition, as contemplated herewith, to said patient.

Generally, the treatment of APDS1 can be ameliorating, curative or prophylactic.

The administration of the engineered HSCs or T-cells, or population of cells, as described herewith, may be carried out in any convenient manner, including injection, transfusion, implantation or transplantation. The engineered HSCs or T-cells, or population of cells, as described herewith may be administered to a patient by intravenous injection.

The administration of the HSCs or T-cells, or population of cells, can consist of the administration of 10⁴-10⁸ gene edited cells per kg body weight, preferably 10⁵ to 10⁶ cells/kg body weight including all integer values of cell numbers within those ranges. The present invention thus can provide more than 10 doses comprising between 10⁴ to 10⁶ gene edited cells per kg body weight originating from a single patient's sampling.

The engineered HSCs or T-cells, or population of cells, as described herewith can be administrated in one or more doses. According to some embodiments, the therapeutic effective amount of cells is administrated as a single dose, especially when permanent engraftment of the engineered HSCs is sought to definitely cure the disease. According to some embodiments, the therapeutic effective amount of cells is administrated as more than one dose over a period time.

As an alternative to permanent HSCs engraftment to cure APDS1, which is a heavy and aggressive treatment for the immune system, the present invention provides with the method of directly engineering T-cells, which are subsequently expanded and frozen for their sequential use. This strategy allows the possibility of multiple re-dosing of the patient over a long period of time, which can span several years. According to some embodiments, the therapeutic effective number of cells is administrated as more than one dose over a period time. Timing of administration is within the judgment of managing physician and depends on the clinical condition of the patient. The dosage administrated will be dependent upon the age, health and weight of the patient receiving the treatment, the kind of concurrent treatment, if any, frequency of treatment and the nature of the effect desired.

### Certain definitions

- Amino acid residues in a polypeptide sequence are designated herein according to the one-letter code, in which, for example, Q means Gln or Glutamine residue, R means Arg or Arginine residue and D means Asp or Aspartic acid residue.
- Amino acid substitution means the replacement of one amino acid residue with another, for instance the replacement of an Arginine residue with a Glutamine residue in a peptide sequence is an amino acid substitution.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- As used herein, "nucleic acid" or "polynucleotides" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Nucleic acids can be either single stranded or double stranded.
- By "mutation" is intended the substitution, deletion, insertion of up to one, two, three, four, five, six, seven, eight, nine, ten, eleven, twelve, thirteen, fourteen, fifteen, twenty, twenty five, thirty, forty, fifty, or more nucleotides/amino acids in a polynucleotide (cDNA, gene) or a polypeptide sequence. The mutation can affect the coding sequence of a gene or its regulatory sequence. It may also affect the structure of the genomic sequence or the structure/stability of the encoded mRNA.
- By "gene" is meant the basic unit of heredity, consisting of a segment of DNA arranged in a linear manner along a chromosome, which codes for a specific protein or segment of protein. A gene typically includes a promoter, a 5' untranslated region, one or more coding sequences (exons), optionally introns, a 3' untranslated region. The gene may further comprise a terminator, enhancers and/or silencers.
- As used herein, the term "locus" is the specific physical location of a DNA sequence (e.g. of a gene, such as the *PIK3CD* gene) in a genome. The term "locus" can refer to the specific physical location of a rare-cutting endonuclease target sequence on a chromosome. Such a locus can comprise a target sequence that is recognized and/or cleaved by a sequence-specific reagent according to the invention. It is understood that the locus of interest of the present invention can not only qualify a nucleic acid sequence that exists in the main body of genetic material (i.e. in a chromosome) of a cell but also a portion of genetic material that can exist independently to said main body of genetic material such as plasmids, episomes, virus, transposons or in organelles such as mitochondria as non-limiting examples.
- By "DNA target", "DNA target sequence", "target DNA sequence", "nucleic acid target sequence", or "target sequence" it is intended a polynucleotide sequence that can be targeted and processed by a sequence-specific reagent according to the present invention. These terms refer to a specific DNA location, preferably a genomic location in a cell, but also a portion of genetic material that can exist independently to the main body of genetic material such as plasmids, episomes, virus, transposons or in organelles such as mitochondria as non-limiting example. The target sequence is defined by the 5' to 3' sequence of one strand of said target. Generally, the target sequence is adjacent or in the proximity of the locus to be processed either upstream (5' location) or downstream (3' location). In a preferred embodiment, the target sequences and the proteins are designed in order to have said locus to be processed located between two such target sequences. Depending on the catalytic domains of the proteins, the target sequences may be distant from 5 to 50 bases (bp), preferably from 10 to 40 bp, more preferably from 15 to 30, even more preferably from 15 to 25 bp. These later distances define the spacer referred to in the description and the examples. It can also define the distance between the target sequence and the nucleic acid sequence being processed by the catalytic domain on the same molecule.
- As used herein, "exogenous" sequence generally refers to any nucleotide or nucleic acid sequence that was not initially present at the selected locus. By opposition "endogenous sequence" means a cell genomic sequence initially present at a locus. An "exogenous" sequence is thus a foreign sequence introduced into the cell, and thus allows distinguishing engineered cells over sister cells that have not integrated this exogenous sequence at the locus.
- By "sequence-specific reagent inducing DNA cleavage" it is meant any active molecule that has the ability to specifically recognize a selected polynucleotide sequence in a genomic locus, preferably of at least 9 bp, more preferably of at least 10 bp and even more preferably of at least 12 bp in length, and that catalyzes the breakage of the covalent backbone of a polynucleotide. Non-limiting examples of a "sequence-specific reagent inducing DNA cleavage" according to the invention include reagents that have nickase or endonuclease activity. The sequence-specific reagent can be a chimeric polypeptide comprising a DNA binding domain and another domain displaying catalytic activity. Such catalytic activity can be for instance a nuclease to perform gene inactivation, or nickase or double nickase to preferentially perform gene insertion by creating cohesive ends to facilitate gene integration by homologous recombination, or to perform base editing as described in Komor et al. (2016) Nature 19;533(7603):420-4.
- The term "endonuclease" generally refers to any wild-type or variant enzyme capable of catalyzing the hydrolysis (cleavage) of bonds between nucleic acids within a DNA or RNA molecule, preferably a DNA molecule. Endonucleases do not cleave the DNA or RNA molecule irrespective of its sequence, but recognize and cleave the DNA or RNA molecule at specific polynucleotide sequences, further referred to as "target sequences" or "target sites". Endonucleases can be classified as rare-cutting endonucleases when having typically a polynucleotide recognition site greater than 10 base pairs (bp) in length, more preferably of 14-55 bp. Rare-cutting endonucleases significantly increase homologous recombination by inducing DNA double-strand breaks (DSBs) at a defined locus thereby allowing gene repair or gene insertion therapies (Pingoud, A. and G. H. Silva (2007). Precision genome surgery. Nat. Biotechnol. 25(7): 743-4.).

- The term "cleavage" refers to the breakage of the covalent backbone of a polynucleotide. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. Double stranded DNA, RNA, or DNA/RNA hybrid cleavage can result in the production of either blunt ends or staggered ends.
- By "vector" is meant a nucleic acid molecule capable of transporting another nucleic acid to which it has been linked. A "vector" in the present invention includes, but is not limited to, a viral vector, a plasmid, a PCR product, a RNA vector or a linear or circular DNA or RNA molecule which may consist of a chromosomal, non-chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available. Viral vectors include retrovirus, adenovirus, parvovirus (e. g. adeno-associated viruses, AAV), coronavirus, negative strand RNA viruses such as orthomyxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).
- By "hematopoietic stem cells" ("HSCs"), it is meant multipotent stem cells derived from the bone marrow that have the capacity to self-renew and the unique ability to differentiate into all of the different cell types and tissues of the myeloid or lymphoid cell lineages, including but not limited to, granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), monocytes (e.g., monocytes, macrophages), dendritic cells, microglia, osteoclasts, and lymphocytes (e.g., NK cells, B-cells and T-cells). It is known in the art that such cells may or may not include CD34+ cells. CD34+ cells are immature cells that express the CD34 cell surface marker. In humans, CD34+ cells are believed to include a subpopulation of cells with the stem cell properties defined above, whereas in mice, HSCs are CD34-. In addition, HSC also refers to long term repopulating HSC (LT-HSC) and short term repopulating HSC (ST-HSC). LT-HSC and ST-HSC are distinguished based on functional potential and on cell surface marker expression. For example, in some embodiments, human LT-HSCs are CD34+, CD38-, CD45RA-, CD90+, CD133+ and ST-HSCs are CD34+, CD38-, CD45RA-, CD90-, CD133-. In addition, ST-HSCs are less quiescent (i.e., more active) and more proliferative than LT-HSCs under homeostatic conditions. However, LT-HSCs have greater self-renewal potential (i.e., they survive throughout adulthood, and can be serially transplanted through successive recipients), whereas ST-HSCs have limited self-renewal (i.e., they survive for only a limited period of time, and do not possess serial transplantation potential). Any of these HSCs can be used in any of the methods described herein. In some embodiments, ST-HSC are useful because they are highly proliferative and thus, can more quickly give rise to differentiated progeny.

- By "long term repopulating HSC" or "LT-HSC" it is meant a type of hematopoietic stem cells capable of maintaining self-renewal and multilineage differentiation potential throughout life. Phenotype markers characteristic for LT-HSCs include, but are not limited to, CD34+, CD38-, CD45RA-, CD90+, and CD133+.
- By "primary cell" or "primary cells" it is meant cells taken directly from living tissue (e.g. biopsy material or blood sample) and established for growth *in vitro* for a limited amount of time, meaning that they can undergo a limited number of population doublings. Primary cells are opposed to continuous tumorigenic or artificially immortalized cell lines. Non-limiting examples of such cell lines are CHO-K1 cells; HEK293 cells; Caco2 cells; U2-OS cells; NIH 3T3 cells; NSO cells; SP2 cells; CHO-S cells; DG44 cells; K-562 cells, U-937 cells; MRC5 cells; IMR90 cells; Jurkat cells; HepG2 cells; HeLa cells; HT-1080 cells; HCT-116 cells; Hu-h7 cells; Huvec cells; Molt 4 cells.
- By "originating from" it is meant that a cell or cells, such as HSCs or T-cells, have been obtained from a patient suffering from APDS1 related to *PIK3CD.* In general, cells are provided from patients through a variety of methods known in the art, as for instance by leukapheresis techniques as reviewed by Schwartz J.et al. [Guidelines on the use of therapeutic apheresis in clinical practice-evidence-based approach from the Writing Committee of the American Society for Apheresis: the sixth special issue (2013) J. Clin. Apher. 28(3): 145-284]. HSCs can be taken from bone marrow, and more particularly from the pelvis, at the iliac crest, using a needle or syringe. Alternatively, HSCs may be harvested from the circulating peripheral blood, while blood donors are injected with a HSC mobilizing agent, such as granulocyte-colony stimulating factor (G-CSF) and/or plerixafor, or CXCL2, that induces cells to leave the bone marrow and circulate in the blood vessels. HSCs may also be harvested from cord blood. HSCs may also be obtained from induced pluripotent stem (iPS) cells derived from the patient.

- "identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 80%, at least 85%, at least 90%, at least 95%, at least 98% or at least 99% identity to specific polypeptides described herein and preferably exhibiting substantially the same functions, as well as polynucleotide encoding such polypeptides, are contemplated. Unless otherwise stated, the present invention encompasses polypeptides and polynucleotides that have the same function and share at least 80 %, generally at least 85 %, preferably at least 90 %, more preferably at least 95 % and even more preferably at least 97 % with those described herein.
- The term "subject" or "patient" as used herein means a human, and more specifically a human suffering from APDS1 related to *PIK3CD.*
- As used herein, the term "about" means plus or minus 10% of the numerical value of the number with which it is being used.

Where a numerical limit or range is stated herein, the endpoints are included. Also, all values and subranges within a numerical limit or range are specifically included as if explicitly written out.

As used herein, the indefinite articles "a" and "an" mean "at least one" or "one or more" unless the context clearly dictates otherwise.

As used herein, the terms "comprising", "including", "having" and grammatical variants thereof are to be taken as specifying the stated features, steps or components but do not preclude the addition of one or more additional features, steps, components or groups thereof.

The above written description of the invention provides a manner and process of making and using it such that any person skilled in this art is enabled to make and use the same, this enablement being provided in particular for the subject matter of the appended claims, which make up a part of the original description.

Having generally described this invention, a further understanding can be obtained by reference to certain specific examples, which are provided herein for purposes of illustration only, and are not intended to limit the scope of the claimed invention.

### EXAMPLES

### Example 1. Materials and Methods for genetic engineering of PIK3CD in T-cells and HSCs

### TALE-nucleases targeting PIK3CD gene

A TALE-nuclease (SEQ ID NO: 40 and SEQ ID NO: 41) was designed to target PIK3CD exon 8 locus. Targeted sequence is:
TGAACGCCGACGAGCGG**atgaaggtggggc**TCCTGGGATAGGTGGGA (SEQ ID NO: 3), with the spacer sequence in bold letters.

A TALE-nuclease (SEQ ID NO: 42 and SEQ ID NO: 43) was designed to target PIK3CD exon 17 locus. Targeted sequence is:
TGATGCACTTGTGCATG**cggcaggaggcctac**CTAGAGGCCCTCTCCCA (SEQ ID NO: 6), with the spacer sequence in bold letters.

A TALE-nuclease (SEQ ID NO: 44 and SEQ ID NO: 45) was designed to target the E1021K mutation in PIK3CD exon 24. The target sequence is:
TGGCACTGGGGAAAACA**gaggaggaggcactgaagcactt**CCGAGTGAAGTTTAACA (SEQ ID NO: 9) with the spacer sequence in bold letters and the E1021K mutation being underlined.

### TALE-Nuclease plasmid production

TALEN backbones were assembled using standard molecular biology and/or microbiology technics such as enzymatic restriction digestion, ligation, bacterial transformation and plasmid DNA extraction. TALE DNA targeting array were assembled and cloned into TALEN backbone using standard molecular biology and/or microbiology technics such as enzymatic restriction digestion, ligation, bacterial transformation (NEB 10-beta competent *E*. *coli for* ccdB selection or NEB stable competent *E*. *coli* for blue/white screening) and plasmid DNA extraction.

### TALEN mRNA production

Sequence verified TALEN plasmids, containing a T7 promoter and a polyA sequence, were linearized with Sapl (NEB) before *in vitro* mRNA synthesis. Small scale mRNA was produced by *in vitro* transcription with NEB HiScribe ARCA (NEB). Alternatively, larger scale mRNA was produced with NEB HiScribe^{™} T7 Quick High Yield RNA Synthesis Kit (NEB). The 5' capping reaction was performed with ScriptCap^{™} m7G Capping System (Cellscript). Antarctic Phosphatase (NEB) was used to treat the capped mRNA and the final cleanups was performed with Mag-Bind TotalPure NGS beads (Omega bio-tek) and Invitrogen DynaMag-2 Magnet (ThermoFisher).

### Targeting-vectors design

Two AAV vectors were designed for APDS1 phenotype correction via genetic engineering in exon 8 **(****Figure 3A** **and** **Figure 4A****).** These vectors contain the PIK3CD exons 8 to 24 cDNA followed by a polyA signal sequence, followed by a C-terminal-deleted LNGFR reporter gene of SEQ ID NO: 21 placed either under an EF1a promoter control and forming a template of SEQ ID NO: 22 or linked to the PIK3CD exons and polyA sequences by a self-cleaving peptide such as P2A and forming a template of SEQ ID NO: 23. Taken together, these sequences form a cassette that is placed between a left- and a right- homologous regions (or "homology arms") allowing the integration of the PIK3CD cDNA and LNGFR coding sequence within the exon 8 that is targeted by the TALE-nuclease, thereby resulting in the expression of a hybrid PIK3CD transcript comprising the endogenous exons 1-7 and the exogenous codon optimized exons 8 to 24 of *PIK3CD.* These AAV vectors are referred to, herewith, as "exon 8 targeting AAV vectors".

The left- and right- homologous regions for targeting exon 8 used herewith have the nucleotide sequence of SEQ ID NO: 12 and SEQ ID NO: 13, respectively.

Two other AAV vectors were designed for APDS1 phenotype correction via genetic engineering in exon 17. These vectors contain the PIK3CD exons 17 to 24 cDNA followed by a polyA signal sequence, followed by a LNGFR reporter gene (C-terminal deleted version) placer either under the control of an EF1a promoter forming a template of SEQ ID NO: 24 or linked to exons 17-24 cDNA by a P2A forming a template of SEQ ID NO: 25. These sequences form a cassette that is placed between a left- and a right- homologous regions allowing the integration of the PIK3CD cDNA and the LNGFR coding sequence within the exon 17 that is targeted by the TALE-nuclease, thereby resulting in the expression of a hybrid PIK3CD transcript comprising the endogenous exons 1-16 and the exogenous codon optimized exons 17 to 24 of *PIK3CD.* These AAV vectors are referred to, herewith, as "exon 17 targeting AAV vectors".

The left- and right- homologous regions for targeting exon 17 used herewith have the amino acid sequence of SEQ ID NO: 14 and SEQ ID NO: 15, respectively.

In general, the left- and right- homologous regions are homologous or identical to the targeted portions of the PIK3CD gene, e.g. homologous or identical with a portion of exon 8 or a portion of exon 17 in the designs described above, or with a portion of exon 24 in the design described below for correction of the E1021K mutation.

Used herewith were the EF1a promoter of SEQ ID NO: 18, the PGK promoter of SEQ ID NO: 19, and the self-cleaving peptide P2A of SEQ ID NO: 20.

### AAV production

Sequence encoding donor templates were synthetized de novo (GenScript) and cloned into AAV production backbone plasmid using standard molecular biology and/or microbiology technics such as enzymatic restriction digestion, ligation, bacterial transformation (NEB stable competent *E*. *coli* for blue/white screening) and plasmid DNA extraction. AAV production was performed by Vigene.

As used herewith, the cells which have been engineered as described herewith are also called "PIK3CD-edited" cells.

### Example 2. TALE-nucleases efficacy

PBMCs were isolated from peripheral blood collected from APDS1 patients using Ficoll-Paque or from cytapheresis rings from Healthy donors using Ficoll-Paque. PBMCs from Healthy Donors or from APDS1 patients were activated with 20 nM phorbol myristate acetate (PMA)/1µM lonomycin for 2 days. After 4 additional days of culture with 20 ng/ml interleulin-2 (IL2), 5×10⁶ T-cells were resuspended in cytoporation buffer T and electroporated using BTX Harvard apparatus in 0.4 cm cuvettes with 15 µg of each TALE-nuclease arm mRNA targeting either exon 8 (SEQ ID NO: 40 and SEQ ID NO: 41) or exon 17 (SEQ ID NO: 42 and SEQ ID NO: 43). After electroporation, the cells were further cultured in medium containing IL2 changed every 2 days. DNA was extracted and the cleavage frequency of the TALE-nuclease was assessed by digital droplet PCR 10 days after electroporation. The quantification of TALE-nuclease efficacy relies on the loss of signal of the probe binding TALE-nuclease cleavage site upon indel and/or missense formation. The list of the primers and probes are provided in Table 1.

Both Healthy Donors ("HD") T-cells and APDS1 patients ("patient") T-cells were targeted by TALE-nuclease targeting exon 8, leading to a mean indel frequencies of 80% and 60%, respectively **(****Figure 2A****).** In addition, both Healthy Donors ("HD") T-cells and APDS1 patients ("patient") T-cells were targeted by TALE-nuclease targeting exon 17, leading to 60% and 20% of indels, respectively **(****Figure 2B****).**

**Table 1: primers and probes for the assessment of indels % by ddPCR**

| | | |
|---|---|---|
| PIK3CDex8 F Primer | SEQ ID NO: 26 | CTGTGGGACTGCCGTGGG |
| PIK3CDex8 R Primer | SEQ ID NO: 27 | TACCCCTCCAGGATACAGCC |
| PIK3CDex8 Probe NHEJ | SEQ ID NO: 28 | CCAGGAGCCCCACCTTCATCCG |
| PIK3CDex17 F Primer | SEQ ID NO: 29 | GCTGAGCTCTCAGAAGACCC |
| PIK3CDex17 R Primer | SEQ ID NO: 30 | GCTCACCAGACTTCAGCCAG |
| PIK3CDex17 Probe NHEJ | SEQ ID NO: 31 | TAGGTAGGCCTCCTGCCGCA |
| RPP30 F Primer | SEQ ID NO: 32 | AGTCTGTCGATGGTGTGCAG |
| RPP30 R Primer | SEQ ID NO: 33 | GGAGGACATTTGAGGAGTGGT |
| RPP30 Probe Reference | SEQ ID NO: 34 | CAGTGGTGTGAGGGAGTTATCACT |

### Example 3. Targeted integration efficacy

### Targeted Integration of a codon optimized PIK3CD cassette with EF1a promoter-LNGFR construct, in PIK3CD exon 8

Preactivated T-cells from Healthy Donors (HD) or from APDS1 patients (patient) were electroporated as described before with 15 µg of exon 8-targeting-TALE-nuclease mRNA and transduced with exon 8-targeting AAV vector comprising exons 8-24 and EF1a-LNGFR construct (described in Example 1) at an MOI of 10⁵. 10 days later, cells were stained with a Fixable viability dye 450 and PE anti-LNGFR antibody (Miltenyi # 130-113-421). Percentage of positive-LNGFR cells, reflecting targeted integration, was assessed by flow cytometry **(****Figure 3B****).** Efficient targeted integration was achieved in HD T-cells (20%) as well as in APDS1 patient T-cells (15%).

### Targeted integration of a codon optimized PIK3CD cassette with P2A-LNGFR construct, in PIK3CD exon 8

Preactivated T-cells were electroporated with 15 µg of exon 8-targeting-TALE-nuclease mRNA and were transduced with the exon 8-targeting AAV vector comprising exons 8-24 and P2A-LNGFR construct (described in example 1) at an MOI of 10⁵. Flow cytometry analysis was carried out 10 days later. 32% and 23% of LNGFR positive cells (reflecting targeted integration) was achieved in HD T-cells and APDS1 patient T-cells, respectively **(****Figure 4B****).**

### Targeted Integration in exon 17

Preactivated T-cells from a single Healthy Donor ("HD") or from a single APDS1 patient ("patient") were electroporated with 15 µg of exon 17-targeting-TALE-nuclease mRNA (described in example 1) and were transduced with one of the exon 17 targeting AAV vectors comprising exons 17-24 and LNGFR cDNA (one where LNGFR expression depends on PGK promoter, the other where LNGFR is linked to the exons 17-24 by P2A, as described in Example 1) (**Figures 5A and 5B**). Flow cytometry analysis was carried out 10 days later. LNGFR positive cells (reflecting targeted integration of the cassette) reached from 8% up to 24% (**Figure 5C**) in HD and APDS1 patient T-cells.

### Example 4: Purification of PIK3CD-edited T-cells

The cells which have been engineered as described herewith are also called "PIK3CD-edited" cells.

### Purification of PIK3CD-edited T-cells comprising a codon optimized PIK3CD-EF1a-LNGFR cassette

PIK3CD-edited T-cells expressing LNFGR were stained using PE anti-LNGFR antibody (Miltenyi, # 130-113-421) and purified using Anti-PE MicroBeads kit (Miltenyi, #130-048-801) on MS column. Cells before purification and cells from the positive and negative fractions were stained with a Fixable viability dye 450 and analyzed by flow cytometry. Both PIK3CD-edited patient T-cells and PIK3CD-edited HD T-cells were successfully purified. Purity reached 78.6% and 85.8%, respectively (**Figure 6A**).

### Purification of PIK3CD-edited T-cells comprising a codon optimized PIK3CD-P2A-LNGFR cassette

PIK3CD-edited T-cells expressing LNFGR were stained using PE anti-LNGFR antibody (Miltenyi, # 130-113-421) and purified using Anti-PE MicroBeads kit (Miltenyi, #130-048-801) on MS column. Cells before purification and cells from the positive and negative fractions were stained with a Fixable viability dye 450 and analyzed by flow cytometry. Both PIK3CD-edited patient T-cells and PIK3CD-editedHD T-cells were successfully purified, and purity reached 97.6% and 93.5% (**Figure 6B**).

### Example 5: Effect of PIK3CD-editing on phosphorylated AKT level

### Effect on phosphorylated AKT level

APDS1 patient T-cells are characterized by a PI3kinase overactivation, which leads to the phosphorylation of downstream effectors including AKT (also called "protein kinase B"). Monitoring the phosphorylation status of AKT (pAKT) is thus a direct readout of PI3kinase activation. In APDS1 patient cells, the basal level of pAKT is high. To ensure that changes in pAKT level are due to PI3kinase, a PI3Kδ inhibitor is included as a control. In addition, an OKT3 antibody is used as a control to induce PI3kinase activation.

PIK3CD-edited T-cells produced in Example 3 and purified as described in Example 4 were either left untouched (basal ø condition), treated with PI3Kδ inhibitor (Calbiochem #IC87114, at a final concentration of 10 nM) or activated with 1µg of OKT3 antibody (Miltenyi #130-093-387). Cells were stained with a fixable viability dye 450 and LNGFR-PE before fixation and permeabilization with methanol. Intracellular staining was carried with APC anti-phospho-AKT antibody (Cell signaling #11962). pAKT expression was assessed on a flow cytometer (**Figure 7** **A and B**).

APDS1 patient T-cells show a high pAKT level: 54% compared to 6% of pAKT positive cells in HD (see MOCK control in **Figure 7A** and **B**). PIK3CD-edited APDS1 patient T-cells which did not express LNGFR showed a reduced level of pAKT, down to 24% or 27% (**Figure 7A** and **Figure 7B****,** LNGFR- condition). This could be explained by the fact that this LNGFR-negative fraction comprises the knock-out of PIK3CD which leads to a reduction in the basal level of pAKT. PIK3CD-edited LNGFR-positive patient T-cells with EF1a-LNGFR construct **(****Figure 6A****)** and P2A-LNGFR construct (**Figure 6B**) showed an even stronger reduction in the basal level of pAKT, down to 15% and 7%, respectively. Importantly, these edited cells can still be activated by OKT3 antibody, up to 47% and 30%, respectively (**Figure 7A** and **Figure 7B****,** LNGFR+ condition).

### Example 6: Effect of PIK3CD editing on cytotoxicity activity

APDS1 patient T-cells were reported to have a reduced cytotoxic activity compared to Healthy Donor T-cells. APDS1 patient T-cells PIK3CD-edited, produced and purified as described in Examples 3 and 4, or non-edited APDS1 patient T-cells ("MOCK") were assessed for cytotoxicity against an EBV-transformed lymphoblastoid cell line (B-EBV) expressing a luciferase reporter gene. Blinatumomab (Invivogen, #bimab-hcd19cd3-1) which is an anti-CD3-CD19 bi-specific T-cell engager was added to promote cytotoxic activity against B-EBVs.

MOCK APDS1 patient T-cells or purified PIK3CD-edited APDS1 patient T-cells (corresponding to the LNGFR-positive fraction obtained in Example 4) were mixed at day 0 with B-EBVs at a ratio of 1 B-EBV: 5 T-cells in complete media supplemented with 5 ng/ml of blinatumomab. During 1 week, media was changed every day and additional B-EBVs and blinatumomab were added. To measure B-EBV expansion, luminescence was assessed every day (**Figure 8A**) and was normalized to conditions of mixed T-cells + B-EBVs without blinatumomab allowing the calculation of the B-EBV expansion index (**Figure 8B**). The purified PIK3CD-edited APDS1 patient T-cells controlled B-EBV expansion better than MOCK APDS1 patient T-cells did (N=2 for each condition). These data strongly suggest that the PIK3CD-edited T-cells are functional and exhibit cytotoxicity activity.

### Example 7. Production and activity of a TALE-nuclease cleaving exon 24 PIK3CD gene, specific to the E1021K mutation

A TALE-nuclease was produced to specifically cleave the target comprising the E1021K mutation in the PIK3CD gene (SEQ ID NO: 9), see also Example 1.

Patient fibroblasts carrying the E1021K mutation were electroporated using the Amaxa nucleofector kit P2 with or without 0.1 µg of each of the mRNAs encoding each of the TALE-nuclease arms (SEQ ID NO: 44 and SEQ ID NO: 45) per 100 000 cells. 7 days later, genomic DNA was extracted and analyzed by ddPCR to quantify indel% using the primers listed in Table 2. The results show that this TALE-nuclease ("TALEN E1021K") was able to produce indels at around 5% (**Figure 9**).

**Table 2: primers and probes for the assessment of indels % by ddPCR**

| | | |
|---|---|---|
| PIK3CDex24 F Primer | SEQ ID NO: 35 | CCGAGTGAAGTTTAACAAAGCCC |
| PIK3CDex24 R Primer | SEQ ID NO: 36 | AGGTGCAGTTCAGGCTCTTC |
| PIK3CDex24 Probe NHEJ5' | SEQ ID NO: 37 | TTTGTTAAACTTCACTCGGAAGTGCTTCAGTGC |
| PIK3CDex24 Probe NHEJ3' | SEQ ID NO: 38 | ACAAAGCCCTCCGTGAGAGCTGGA |
| PIK3CDex24 Reference Probe | SEQ ID NO: 39 | TGTTTGCAGGACTCCCTGGCACT |

### Example 8. TALE-Nucleases' efficacy in CD34+ HSPCs

CD34+ HSPCs derived from G-CSF mobilized peripheral blood obtained from healthy donors were purchased from a commercial provider. HSPCs were cultured in SFEMII media supplemented with cytokines CD34+ supplement for 48h prior to gene editing.

10⁶ cells were resuspended in BTXpress buffer and electroporated using BTX Harvard apparatus in 0.4 cm cuvettes with increasing quantities of each TALE-nuclease arm mRNAs targeting PIK3CD exon 8 described in Example 1 (SEQ ID NO: 40 and SEQ ID NO: 41). Genomic DNA was extracted and the cleavage frequency of the TALE-nuclease was assessed by digital droplet PCR (ddPCR) five days after electroporation. The quantification of TALE-nuclease efficacy relies on the same strategy used in T-cells described in Example 2: the loss of signal of the probe binding TALE-nuclease cleavage site upon indel and/or missense formation. The list of the primers and probes are provided in Table 1.

A dose dependent cutting efficiency was observed and the highest Indels frequencies were obtained at the 15 and 20 µg/arm doses (83% and 86% respectively) (**Figure 10A**).

### Example 9. Targeted integration efficacy for PIK3CD correction in CD34+ HSPCs

HSPCs were cultured and electroporated as described before, using increasing doses of TALE-nuclease mRNA. 15' after electroporation cells were transduced with a 5e4 MOI dose of AAV vector delivering the DNA homology donor cassette EF1a-LNGFR described in Example 1 (SEQ ID NO: 22).

Five days after electroporation cells were used either for genomic DNA extraction followed by ddPCR or for FACS analysis to measure the efficiency of targeted integration. The quantification of targeted integration efficacy by ddPCR relies on the acquisition of signal from the probe discriminating the codon optimized cassette from the endogenous sequence. The list of the primers and probes are provided in Table 1. Targeted integration was achieved at all TALE-Nuclease mRNA doses from 6% to 8% of edited alleles as measured by ddPCR (**Figure 10B**). For FACS analysis, cells were stained with a Fixable viability dye 480 (eBioscience #65-0865-18) and PE anti LNGFR antibody (Miltenyi # 130-113-421). LNGFR expression confirmed the efficient targeted integration with frequencies spanning from 3.7% to 5.3% reached at highest TALE-nuclease mRNA dose (**Figure 10C**).

Three different AAV MOIs (1e4, 5e4 and 1e5) were tested at the 15µg/arm TALE-nuclease mRNA dose and targeted integration was assessed by ddPCR and LNGFR FACS analysis.

Efficient targeted integration was obtained at all MOIs tested and the highest targeted integration efficiency was observed at the 5e4 MOI as measured by ddPCR or LNGFR expression by FACS (8% and 4.7% respectively) (**Figure 11**).

As high AAV MOI coupled to 15 µg/arm TALE-nuclease mRNA dose results in decrease in CD34+ viability and proliferation (**Figure 12**), the 5e4 MOI was chosen as optimal considering editing efficiencies and toxicity in CD34+ cells and tested in two further donors coupled to 5 µg dose of TALE-Nuclease mRNA.

Efficient targeted integration was achieved in HSPCS from two healthy donors with a frequency of up to 11.6 % edited alleles measured by ddPCR and up to 10.8 % LNGFR expression measured by FACS (**Figure 13**).

### Example 10: Purification of PIK3CD-edited HSPCs expressing LNGFR

PIK3CD-edited HSPCs expressing LNFGR were stained and purified using CD271 MicroBeads kit (Miltenyi #130-099-023) on MS column. Cells from the positive and negative fractions were stained with a Fixable viability dye 480 (eBioscience #65-0865-18) and a PE anti-LNGFR antibody (Miltenyi, # 130-113-421) then analyzed by flow cytometry.

Edited HSPCs were successfully purified, and purification yield assessed at 3 days after sorting was 74% with only 3% of LNGFR-expressing cells remaining in the negative fraction **(****Figure 14****).**

## Claims

1. An engineered hematopoietic stem cell (HSC) or T-cell originating from a patient suffering from Activated PI3kinase Delta Syndrome type 1 ("APDS1"), comprising an exogenous polynucleotide sequence comprising the sequence of a functional *PIK3CD* gene ("PIK3CD sequence") or a portion thereof of at least 100 nucleotides, said exogenous polynucleotide sequence being integrated in the cell's genome in an endogenous *PIK3CD* allele comprising at least one mutation causing APDS1, thereby resulting in the presence, in said cell's genome, of a nucleic acid sequence encoding a functional PI3Kδ protein.

2. The engineered cell according to claim 1, wherein said exogenous polynucleotide sequence comprises a PIK3CD sequence, or portion thereof, encoding the amino acid sequence of SEQ ID NO: 46 or a portion thereof of at least 35 amino acids.

3. The engineered cell according to claim 1 or 2, wherein said exogenous polynucleotide sequence comprises a PIK3CD sequence, or portion thereof, comprising (i) SEQ ID NO: 1 or a portion thereof of at least 100 nucleotides and wherein said exogenous polynucleotide sequence is integrated within Exon 8 of said endogenous *PIK3CD* allele in the cell's genome, or (ii) SEQ ID NO: 2 or a portion thereof of at least 100 nucleotides and wherein said exogenous polynucleotide sequence is integrated within Exon 17 of said endogenous *PIK3CD* allele in the cell's genome.

4. The engineered cell according to any one of claims 1 to 3, wherein said exogenous polynucleotide sequence further comprises a sequence encoding a surface marker such as LNGFR, EGFRt, appropriate for purification and/or detection of said engineered cell.

5. A population of cells comprising engineered cells according to any one of claims 1 to 4.

6. A TALE-Nuclease heterodimer targeting a polynucleotide sequence comprised within the *PIK3CD* gene, comprising:
a) a first monomer comprising the amino acid sequence of SEQ ID NO: 40 or a variant thereof comprising an amino acid sequence having at least 80% sequence identity with SEQ ID NO: 40, and a second monomer comprising the amino acid sequence of SEQ ID NO: 41 or a variant thereof comprising an amino acid sequence having at least 80% sequence identity with SEQ ID NO: 41, wherein the heterodimer targets the sequence of SEQ ID NO: 3;
b) a first monomer comprising the amino acid sequence of SEQ ID NO: 42 or a variant thereof comprising an amino acid sequence having at least 80% sequence identity with SEQ ID NO: 42, and a second monomer comprising the amino acid sequence of SEQ ID NO: 43 or a variant thereof comprising an amino acid sequence having at least 80% sequence identity with SEQ ID NO: 43, wherein the heterodimer targets the sequence of SEQ ID NO: 6; or
c) a first monomer comprising the amino acid sequence of SEQ ID NO: 44 or a variant thereof comprising an amino acid sequence having at least 80% sequence identity with SEQ ID NO: 44, and a second monomer comprising the amino acid sequence of SEQ ID NO: 45 or a variant thereof comprising an amino acid sequence having at least 80% sequence identity with SEQ ID NO: 45, wherein the heterodimer targets the sequence of SEQ ID NO: 9.

7. An isolated nucleic acid or vector encoding a TALE-Nuclease heterodimer according to claim 6.

8. An isolated nucleic acid or a vector comprising an exogenous polynucleotide sequence as defined in any one of claims 1 to 4, wherein said exogenous sequence is placed between a left homologous region at its 5' end and a right homologous region at its 3' end, homologous regions being relative to the targeted portion of the *PIK3CD* gene.

9. A pharmaceutical composition comprising an engineered cell according to any one of claims 1 to 4, or a population of cells according to claim 5, and a pharmaceutically acceptable excipient and/or carrier.

10. An engineered cell according to any one of claims 1 to 4, a population of cells according to claim 5, or a pharmaceutical composition according to claim 9, for use in the treatment of Activated PI3kinase Delta Syndrome type 1.

11. A kit comprising:
(i) at least one isolated nucleic acid or vector comprising an exogenous polynucleotide sequence according to claim 8, and
(ii) at least one isolated nucleic acid or vector encoding a TALE-nuclease heterodimer according to claim 6.

12. A method of engineering HSCs or T-cells having a non-functional endogenous *PIK3CD* allele comprising at least one APDS1-associated mutation in said cells, said method comprising introducing into said cells:
(i) at least one sequence specific reagent inducing DNA cleavage that is capable of specifically targeting and cleaving a sequence within an endogenous *PIK3CD* locus, in at least a non-functional *PIK3CD* endogenous allele, in said cells; and
(ii) at least one isolated nucleic acid or vector comprising an exogenous sequence as defined in any one of claims 1 to 4, wherein said exogenous sequence is placed between a left homologous region at its 5' end and a right homologous region at its 3' end, homologous regions being relative to the targeted portion of the *PIK3CD* gene and, optionally, wherein said exogenous sequence further comprises a nucleic acid sequence encoding a surface marker;
whereby engineered PIK3CD-edited HSCs or T-cells are obtained, which comprise a non-functional *PIK3CD* allele that has been disrupted and comprise a nucleic acid sequence encoding a functional PI3Kδ protein.

13. The method according to claim 12, wherein said HSCs or T-cells to be engineered are isolated from a tissue sample from a human patient suffering from Activated PI3kinase Delta Syndrome type 1.

14. A method of purifying engineered PIK3CD-edited HSCs or T-cells originating from a patient suffering from APDS1, comprising:
(i) providing a population of cells comprising the engineered HSCs or T-cells according to claim 4 or the population of cells obtained by the method according to claim 12;
(ii) binding the engineered HSCs or T-cells with a binding agent specific for said surface marker, wherein said binding agent is fixed to a support; and
(iii) eluting the engineered HSCs or T-cells from the support;
wherein the eluted product is enriched in PIK3CD-edited HSCs or T-cells.

15. An engineered hematopoietic stem cell (HSC) or T-cell originating from a patient suffering from APDS1, wherein the endogenous *PIK3CD* allele comprising at least one APDS1 associated mutation has been cleaved by a sequence specific reagent, thereby resulting in the expression of only a functional *PIK3CD* gene in said engineered cell.
